# EUROPEAN PATENT SPECIFICATION

(11) **EP 0 774 251 B1**
(45) Date of publication and mention of the grant of the patent: **14.07.2004**
(21) Application number: 96307928.0
(22) Date of filing: 31.10.1996
(51) Int. Cl.: A61K 31/047, A61P 27/02

(54) **3R-3'R stereoisomer of zeaxanthin for treating macular degeneration in humans**
3R-3'R Diastereoisomer von Zeaxanthin zur Behandlung von der Degeneration der Macula
3R-3'R diastéréoisomère de la zéaxanthine pour le traitement la dégénérescence maculaire

(30) Priority: 31.10.1995 US 550665; 31.10.1995 US 551153; 31.10.1995 US 551166
(43) Date of publication of application: 21.05.1997
(73) Proprietor: ZeaVision, L.L.C., Chesterfield, MO 63017 (US)
(72) Inventor: Garnett, Kevin M., St.Louis, Missouri 63104 (US); Gierhart, Dennis L., High Ridge, Missouri 63049 (US); Guerra-Santos, Luis H., Ballwin, Missouri 63011 (US)
(74) Representative: Nicholls, Kathryn Margaret

(56) References cited:
- EP-A- 0 393 690
- WO-A-91/03571
- WO-A-96/40092
- US-A- 5 290 605
- AMERICAN JOURNAL OF CLINICAL NUTRITION, vol. 62, no. 6, December 1995, pages 1448S-1461S, XP000603641 SNODDERLY D M: "EVIDENCE FOR PROTECTION AGAINST AGE-RELATED MACULAR DEGENERATION BY CAROTENOIDS AND ANTIOXIDANT VITAMINS"
- JAMA THE JOURNAL OF THE AMERICAN MEDICAL ASSOCIATION, vol. 272, no. 18, 9 November 1994, pages 1413-1420, XP000603671 SEDDON J M ET AL: "DIETARY CAROTENOIDS, VITAMINS A,C, AND E, AND ADVANCED AGE-RELATED MACULAR DEGENERATION"
- INVESTIGATIVE OPHTHALMOLOGY AND VISUAL SCIENCE, vol. 36, no. 4, 15 March 1995, page s892 XP002016871 LANDRUM, J.T. ET AL: "Macular pigment stereomers in individual eyes: A comparison between normals and those with age-related macular degeneration"
- JOURNAL OF THE AMERICAN OSTEOPATHIC ASSOCIATION, vol. 95, no. 1, January 1995, page 26 XP000603675 SEDDON J M: "DO ANTIOXIDANTS PREVENT OR RETARD THE ONSET OF AMD?"
- INVESTIGATIVE OPHTHALMOLOGY AND VISUAL SCIENCE, vol. 34, no. 6, May 1993, pages 2033-2040, XP000645672 BONE, R.A. ET AL: "Stereochemistry of the human macular carotenoids"
- NUTRITION REVIEWS, vol. 38, no. 11, November 1980, pages 384-386, XP000603663 "THE EFFECT OF A DIETARY LACK OF XANTHOPHYLL ON THE EYE OF THE MONKEY"
- INVESTIGATIVE OPHTHALMOLOGY AND VISUAL SCIENCE, vol. 29, no. 6, 1988, pages 850-55, XP000645674 HANDELMAN, G.J. ET AL: "Carotenoids in the human macula and whole retina"
- INVESTIGATIVE OPHTHALMOLOGY AND VISUAL SCIENCE, vol. 36, no. 4, 1995, page s233 XP000645697 HAMMOND, B.R. ET AL: "tHE RELATIONSHIP BETWEEN CIGARETTE SMOKING AND PEAK MACULAR PIGMENT DENSITY"
- Declarations of Kevin Garnett; Applied Food Biotechnology
- 'Age-Related Eye Disease Study' CELEBRATING VISION RESEARCH 1993, pages 7 - 9
- 'The Biochemistry of Carotenoids, Vol.1: Plants', 1980, CHAPMAN HALL, LONDON
- 'Report of the retinal diseases panel' VISION RESAERCH, A NATIONAL PLAN: 1999-2003 pages 13 - 38
- MARES-PERLMAN J.A. ET AL ARCH. OPHTHALMOL. vol. 113, 1995, pages 1518 - 1523

## Description

### BACKGROUND OF THE INVENTION

This invention is in the field of biochemistry, and relates to a certain isomer of a yellow pigment called zeaxanthin (abbreviated as ZX). If administered to humans as a drug or vitamin, this pigment can treat or prevent a disease called macular degeneration, which damages the retina and can cause blindness.

The retina is the tissue that lines the back of the eyeball. It is complicated, and contains a dozen distinct layers. It is described and illustrated in many medical textbooks, such as Gittinger 1988, and Vaughn and Asbury 1992 (full citations are listed below).

A special circular region called the *macula,* about 1 to 1.5 millimeters in diameter in humans, is located in the middle of the retina. The macula has two characteristics that distinguish it from the rest of the retina. First, the macula contains relatively few rods; most of its photoreceptors are cone-shaped (in the fovea, at the very center of the macula, there are no rods at all). And second, the macula has a distinct yellow color, caused by two pigments called *lutein* and zeaxanthin. Both belong to a class of molecules called "carotenoids". The chemistry of these carotenoid pigments is discussed below, after the summary of macular degeneration.

### Macular Degeneration

"Macular degeneration" refers to any condition that involves progressive damage to the retinal cells or photoreceptor cones in the yellow macular region in the center of the retina. It is described and illustrated in articles and texts such as Taylor 1993, Gittinger 1988, and Vaughan and Asbury 1992.

There are several types of macular degeneration. The most common type is called "age-related macular degeneration", usually abbreviated as AMD (or as ARMD in some articles). AMD can damage the eyesight in ways ranging from slight loss, to total blindness.

There are two forms of AMD, often referred to as the "wet" and "dry" forms. The wet form involves aggressive growth of capillaries and other blood vessels into the retina, to a point where the blood vessels disrupt and destroy the proper organization of the retinal layers. Although this form of AMD can sometimes be treated by using a laser to close off the newly forming blood vessels, laser treatment can only retard blood vessel growth for a while, and it usually cannot prevent the eventual loss of almost all vision; wet AMD almost always leads eventually to total or nearly-total blindness. The wet form occurs in only about 5 to 10 percent of patients suffering from AMD.

The other form of AMD is called "dry" AMD. Since it occurs in at least 90% of all cases, it is often referred to simply as AMD. Although this form of AMD usually does not cause total blindness, it can cause severe damage to a patient's eyesight, and renders a patient unable to read or identify well-known objects such as the faces of friends or relatives. As such, it often leads to functional blindness, rendering people unable to drive or walk safely in public, and unable to carry out normal activity.

There are also various diseases that involve macular degeneration as a symptom, including Stargardt's disease, Best's disease, Batten's disease, Sjogren-Larsson syndrome, cone-rod dystrophy, and ovine ceroid lipofuscinosis. Articles describing each of these are cited in Dorey et al 1993. In addition, other diseases which involve lysosomal storage problems (such as Tay-Sach's disease) or progressive nerve cell degenerative (such as Alzheimer's disease) are also correlated with macular degeneration.

Many of these diseases have genetic components, as evidenced by inheritance; some of the genes which cause these diseases have been isolated, and genetic screening tests can indicate whether a person has the defective gene. Any person who carries or likely carries such a gene, as evidenced by genetic testing or family history, is at elevated risk of macular degeneration.

By slowly robbing people of their eyesight, AMD inflicts terrible suffering on its victims. It costs billions of dollars each year, both in loss of productivity, and in the heavy burdens on family members, insurance providers, social agencies, and others who must provide or help pay for medical care and other assistance for people who suffer from blindness or severe damage to their eyesight.

In view of the problems caused by AMD, scientists and doctors have been searching for decades for ways to treat or prevent blindness and other vision loss caused by macular degeneration. However, despite all those efforts for more than half a century, no effective treatments are available today.

### Diagnosis: Drusen and Lipofuscin

Macular degeneration is usually diagnosed by special photographs of the retina. In one type of diagnostic procedure, a physician injects a fluorescent drug into a patient, gives the drug time to circulate throughout the patient, and then takes a magnified photograph of the retina, called an angiogram. The physician then analyzes the photograph to determine the presence and concentration of either or both of two types of cellular debris.

One type of cellular debris, which has been known and studied for several decades, is called *drusen.* It comes in two distinct forms. A small quantity of *hard* drusen (small particles with a diameter of less than 63 micrometers) is usually present in the eyes of anyone over 40 years old. Unless present at abnormal levels, hard drusen does not indicate retinal damage.

By contrast, a significant quantity of larger *soft* drusen deposits (also called wet drusen) indicates that substantial retinal damage has occurred or commenced, because large blobs of soft drusen can disrupt and disorganize the retinal layers, and can prevent the retinal cells from receiving proper nutrition from the blood. A patient whose retinas contain a significant quantity of *soft* drusen is usually regarded as suffering from macular degeneration.

The other type of retinal debris that is usually present in patients suffering from macular degeneration is called *lipofuscin.* The correlation between lipofuscin and AMD has become clear only recently (e.g., Weiter et al 1988, and Dorey et al 1993).

### Carotenoid Chemistry

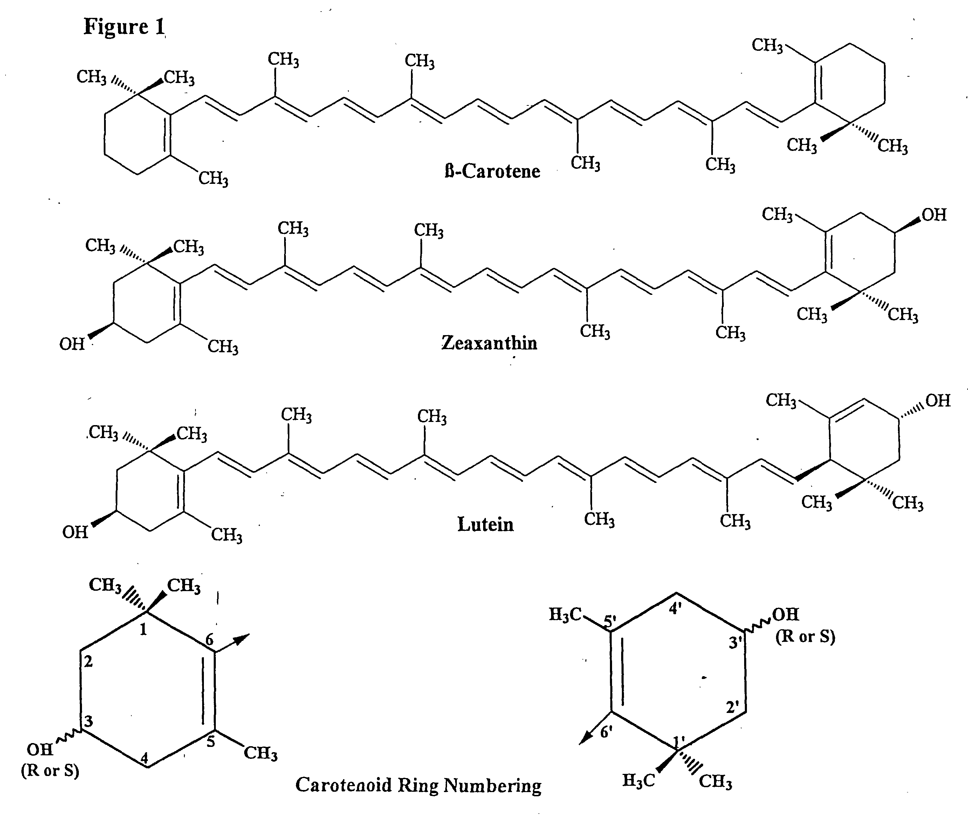

"Carotenoids" includes a large class of molecules; more than 600 carotenoids have been identified in nature. These molecules share several traits, including:
1. Carotenoids are created by coupling together molecules of isoprene, a 5-carbon molecule. Because the building block contains 5 carbon atoms, most carotenoids contain multiples of 5 carbon atoms.
2. Carotenoids have multiple unsaturated bonds. This allows them to absorb high-energy light waves in the blue and near-ultraviolet regions of the spectrum.
3. Because carotenoids absorb wavelengths in the blue and near-ultraviolet region of the spectrum, without absorbing longer wavelengths in other regions of the spectrum, carotenoids usually have yellow, orange, brown, or red colors. The name "carotenoid" derives from carrots; the first known carotenoids were identified as the pigments that give carrots an orange color. The color caused by carotenoids in solution will depend on various factors, including concentration and the presence of other chemicals.
4. Carotenoids have "conjugated" double bonds. This indicates that the double bonds alternate with single bonds, so that each carbon atom in a chain is double-bonded to one other carbon atom, but no carbons are double-bonded to two other carbons. This arrangement can be understood by considering Figure 1, which shows the structures of ß-carotene, ZX, and lutein.

Different carotenoids have different levels of conjugation, and in general, a more highly conjugated molecule will provide better protection against "phototoxic" damage by high-energy light radiation. For example, in the three carotenoids shown in Fig. 1, the entire straight chain portion of each is conjugated, with alternating double and single bonds. In β-carotene and ZX, the conjugation extends to the first bonds in both end rings. By contrast, lutein has a lower conjugation level, since the double bond in one of its end rings does not have the proper placement for complete conjugation. The only difference between ZX and lutein is in the location of the double bond in one (but not both) of the end rings.

Because carotenoids are designed and selected (through evolution) to absorb the potentially harmful energy of blue and near-ultraviolet light, they are used as protective pigments in nature. They are found extensively in plants, since one of the main goals of plants is to absorb as much sunlight as possible, while minimizing cellular damage caused by blue, ultraviolet, and near-ultraviolet radiation. Ultraviolet radiation damage to plants is a major problem, and carotenoids help minimize it.

Because carotenoids are well suited for protecting against phototoxic damage, animals have also acquired (through evolution) ways to utilize carotenoids as photo-protective pigments. Since animals cannot synthesize carotenoids in their bodies, they must ingest carotenoids (or carotenoid precursors) from plant sources. β-carotene is one example; mammals must obtain it from plants, or from meat. Once inside a mammalian body, β-carotene is converted into other molecular forms, including Vitamin A (retinol), which is formed by splitting β-carotene into two halves.

Carotenoids are divided into two main classes, carotenes and xanthophylls. Carotenes do not contain any oxygen; they are true hydrocarbons, formed from only carbon and hydrogen. By contrast, xanthophylls (such as ZX and lutein) also contain oxygen.

Fig. 1 shows the numbering of the carbon atoms in the left and right end rings of ZX. By convention, the carbon atoms in the left end ring are numbered 1 through 6, while the carbon atoms in the right end ring are referred to by "prime" numbers, such as the 3' carbon (pronounced "three prime"). Since ZX is completely symmetrical with regard to the left and right ends, the terms "left" and "right" are merely a convention, to simplify discussion. However, it should be noted that lutein is not symmetrical; the position of the double bond in the "left" ring is not the same as the placement of the double bond in the "right" ring.

Since ZX is formed by adding two hydroxy groups to β-carotene, at the #3 carbon atoms on both ends, its chemical name is 3,3'-dihydroxy-β-β-carotene; some chemists refer to it as carotene-diol. This molecule was given the name "zeaxanthin" because it was first identified as the pigment which gives corn its yellow color, and the scientific name for corn is *Zea mays.*

As noted above, more than 600 carotenoids have been identified in nature. Several dozen are important in biochemistry and commerce. ZX and lutein are especially important in this invention, because they are present in the retinas of mammals and most other animals.

Lutein is commercially important, since it is widely fed (in the form of plant extracts, mainly from marigolds) to chickens, to give their skin and egg yolks more yellow coloring, which appeals to shoppers and consumers.

ZX can have the same effect and is more potent than lutein, but ZX sources have been too expensive for use in poultry feeds. US patents 5,308,759 and 5,427,783 were intended to address the problem of ZX being too expensive for use in animal feed. These patents relate to using bacteria to produce ZX in commercial quantities, so it can be fed to poultry and fish.

In addition to plants, some carotenoids are synthesized by certain bacteria. These bacteria evolved in locations exposed to direct sunlight, and their carotenoids perform the same photo-protective role as in plants. Items describing carotenoids from bacteria include McDermott et al 1972, US patent 5,429,939 (Misawa et al 1995), and other articles cited in those references.

US patent 5,429,939 (Misawa et al 1995) lists the DNA sequences of a number of genes which encode enzymes involved in the biosynthesis of various carotenoids, including ZX. The role of each major gene (including crtE, crtB, crtI, crtY, and crtZ) in creating ZX is also described. Cells containing plasmids containing these genes were deposited with the American Type Culture Collection (such as *Erwinia uredovora* ATCC 19321, and *Erwinia herbicola* ATCC 39368), and with the Fermentation Research Institute in Japan (e.g., *E. coli* FERM BP 2377).

### Zeaxanthin Stereochemistry and Isomers

An important aspect of carotenoid chemistry involves "stereochemistry" and "stereoisomers". This topic is explained in any college textbook on organic chemistry.

Since both the #3 and #3' carbon atoms in ZX are chiral, it has four possible stereoisomers. In the 3R-3'R isomer, the #3 and #3' carbon atoms both have R configurations. In the 3S-3'S isomer, the #3 and #3' carbon atoms both have S configurations. For convenience, these two stereoisomers are referred to herein as the R-R isomer, and the S-S isomer.

The third and fourth isomers are the two "mixed" or "meso" (one R and one S) isomers: the 3R-3'S isomer, and the 3S-3'R isomer. Since ZX is completely symmetric about its midpoint, these two isomers are identical in every respect; if the 3R-3'S isomer is drawn on paper, rotating the paper will turn it into the 3S-3'R isomer. In effect, a single "meso" isomer is formed by both the S-R and R-S isomers.

If standard chemical synthesis techniques are used to make ZX, each of the four possible isomers will be present as about 25% of the total. However, since the S-R and R-S isomers are actually identical, their "meso" isomer will form 50% of the total, while the R-R and S-S isomers will be present at about 25% each. A mixture of all three stereoisomers is called a "racemic" mixture.

However, the cell and enzyme specificities of carotenoids in retinal tissue are extremely precise, and different isomers or stereoisomers are not interchangeable. Lutein and ZX are treated by retinal cells as completely different and distinct molecules, even though they would be regarded as isomers of each other under conventional chemical terminology (since they have identical numbers of carbon, hydrogen, and oxygen atoms).

Because of biological factors, the only isomers that are relevant herein are stereoisomers. Any reference herein to an "isomer" of zeaxanthin refers to a particular stereoisomer of ZX, and does not include lutein. Lutein and ZX are regarded as completely different carotenoids.

Stereoisomeric differences in carotenoids which might appear small, subtle, and insignificant are, in fact, extremely important when it comes to retinal tissue. Apparently, the only ZX stereoisomer that is properly taken up and used by human retinal cells is the R-R isomer (the 3R-3'R stereoisomer).

There have been reports that trace quantities of the meso (R-S) isomer of ZX have been found in retinal tissue. However, these trace quantities are probably attributable to certain molecular conversions that can occur spontaneously under some conditions, leading to formation of meso-zeaxanthin from lutein precursors (Bone et al 1993 and 1994).

In a laboratory, the stereoisomers of ZX can be distinguished from each other using methods such as chiral column chromatography (Bone et al 1993) or circular dichroism analysis (Britton 1994).

### Zeaxanthin and Lutein in the Macula

By 1970, the role of carotenoids in protecting plants against photo-toxic damage was well-known. It was also known that carotenoids were present in animal tissue, and that all carotenoids in animals were originally derived from plants, since animals cannot synthesize carotenoids. Based on that information, various articles pointed out similarities between carotenoids in animals and plants, and concluded that carotenoids protect against phototoxic damage in animals.

After the photo-protective role of carotenoids in animals was recognized, researchers began studying the chemistry and roles of carotenoids in the retina. One line of experiments involved tests in which lab animals were fed diets that did not contain any carotenoids, made from grains or seeds that do not contain carotenoids (such as milo seeds). The results indicated that retinas in carotenoid-deprived lab animals did not develop any yellow macular areas, and these retinas contained abnormally high levels of soft drusen, indicating retinal damage (Malinow et al 1980, Kirschfeld 1982, Ham et al 1984, and Snodderly et al 1984). In view of these findings, researchers suggested that carotenoids appeared to be essential to healthy retinas. These researchers were gathering molecular support for the old wisdom that carrots and leafy green vegetables are good for the eyes. However, scientists did not yet know whether the yellow pigments in the macula had to be ingested in final form, or whether they could be synthesized in animals by using other precursors, such as beta-carotene or lycopene.

Lutein was identified in 1949 as one of the yellow pigments in the macula (Wald 1949). ZX was not identified as the other macular pigment until many years later (Bone et al 1985). Articles which summarize what was known about the macular pigments by the mid or late 1980's include Handelman and Dratz 1986, Werner et al 1987, Pease et al 1987, Haegerstrom-Portnoy 1988, and Handelman et al 1988. Among other things, these articles established that ZX (which is fully conjugated, and which therefore offers somewhat better protection than lutein against damage caused by light energy) is the predominant pigment in the fovea, a small region at the very center of the macula. The quantity of ZX gradually decreases, and the quantity of lutein increases as one travels concentrically away from the fovea, toward the outer edges of the macula, so that at the outer periphery of the macula, lutein is the dominant yellow pigment. More recent articles that address various aspects of retinal aging and damage, and which focus specifically on carotenoids as protective agents in the retina, include Sperduto et al 1990, Gerster 1991, Schalch 1992, and Seddon et al 1994.

In summary, it has been known for more than 10 years that lutein and ZX are the two pigments in the macula, and scientists have been speculating for more than a decade that these pigments may help protect the macula against phototoxic damage.

However, despite the fact that these discoveries and suggestions were all made more than 10 years ago, no one has yet developed any type of drug, any nutritional or dietary supplement, or other form of treatment which is known to be effective in significantly preventing or retarding (let alone reversing) the gradual progression of macular degeneration.

The previous sentence needs to be qualified somewhat, since β-carotene, vitamin A, and vitamin E are all known to have some level of beneficial effect in helping to protect retinal tissue (see, e.g., US patent 5,310,764, Baranowitz et al 1994, and the two articles by the Eye Disease Case Control Study Group, cited below). These patents and articles have claimed or suggested that β-carotene, vitamin A, and vitamin E can have a detectable effect in preventing or reducing the damage associated with macular degeneration.

Such claims may be true, due to the general antioxidant roles of carotenoids, vitamin A, and vitamin E. However, it is also sadly true that the benefits provided by β-carotene, vitamin A, and vitamin E in the retina are very limited, and do not rise to the level of effective treatments. For all practical purposes, macular degeneration is unpreventable, unstoppable, and irreversible. Any broad-spectrum anti-oxidants (such as β-carotene, vitamin A, and vitamin E) are merely palliative measures. Since nothing truly effective has been available, those vitamins have been used (with very limited and unsatisfactory success) to try to slow down the relentless damage caused by macular degeneration.

As a matter of prior art, it should also be noted many health food stores sell carotenoid preparations that are labelled as being beneficial to the eyes and eyesight. That labelling claim on carotenoid mixtures may be reasonable, since (as noted above) β-carotene and vitamin A are known to be useful as general anti-oxidants. However, no commercially available carotenoid mixture contains more than extremely small "trace" quantities of ZX. The great majority of the carotenoids in the carotenoid mixtures that are sold in health food stores are non-zeaxanthin carotenoids (mainly β-carotene and vitamin A).

The positions and research goals of several important government agencies and research consortia are also worth careful attention. In the United States of America, the National Institutes of Health (acting through the National Eye Institute (NEI) and the National Advisory Eye Council) issued two recent reports entitled, "Vision Research: A National Plan 1994-1998," NIH Publication No. 93-3186 (1994; see pages 55-65 in particular), and "Age related eye disease study," NIH Publication 93-2910 (1993). Both publications, and the research they describe, focus on β-carotene (rather than ZX) as the compound which holds the greatest promise for treating AMD. To the best of the Applicants' knowledge and belief, after discussing the subject with officials of the NEI, neither the NEI nor any other organization affiliated with the National Institutes of Health is willing to fund, or has recently funded, any research on zeaxanthin as a potential treatment for AMD. Instead, the NIH and other tax-funded organizations are allocating millions of dollars to carry out research on β-carotene as the most promising candidate agent for treating or preventing AMD.

Other prominent researchers who deserve careful attention belong to the "Eye Disease Case Control Study Group". This group recently published two articles entitled, "Antioxidant status and neovascular age-related macular degeneration," Arch. Ophthalmol. 11: 104-109 (1993), and "Risk factors for neovascular age-related macular degeneration," Arch. Ophthalmol. 10: 1701-1708 (1992). As in the official NIH reports, neither of these articles teaches or suggests the use of zeaxanthin as a drug for treating AMD, and this consortium also has declined or refused to fund any research into ZX as a possible agent for treating or preventing AMD.

It should also be noted that there is great interest in carotenoids for treating or preventing cancer (beginning with Peto et al 1981), and for preventing cholesterol formation and reducing plaque deposits inside arteries (Jialal et al 1991). There is an enormous body of scientific literature dealing with the various activities of carotenoids, and there has been great interest in ways to chemically synthesize carotenoids, including lutein and ZX. However, despite all the carotenoid studies and synthesis efforts of the past decades, no one has yet reported any effective method of treating or preventing macular degeneration. In view of the enormous cost and suffering inflicted on victims and society by macular degeneration, that is a huge problem, which must be addressed.

Accordingly, the subject invention offers a potentially important breakthrough in providing both (1) a safe and effective drug, for treating patients who have been diagnosed as suffering from macular degeneration, and (2) a nutritional supplement, comparable to a vitamin pill, which can be taken by anyone who wants to reduce the risk that he or she may suffer from macular degeneration after reaching or passing middle age.

### Synthesis of Lutein and Zeaxanthin: Prior Art

Various items of prior art describe methods for creating ZX. These items can be grouped into two categories: fermentation methods, in which microbes play a key role in the manufacturing process; and non-fermentation synthesis methods, in which purely chemical reactions are used. Most items of prior art suggested that the ZX should be used for known purposes, such as additives in poultry or fish feeds, to darken the color of the meat and make it more attractive. Apparently, none of those efforts ever resulted in any production or sale of ZX in commercial quantities.

As of October 1995, the only way to purchase ZX, either in purified form or in semi-concentrated form where ZX comprises more than about 5% by weight, requires the purchase of milligram quantities of ZX, from specialty chemical companies such as Atomergic Chemicals Corporation (Farmingdale, NY) or Spectrum Chemical Manufacturing Company (Gardena, CA). The 1995 prices of purified ZX from these specialty manufacturers, in synthetic racemic mixtures that contain the undesired S-S and S-R isomers, ranges from about $90 to about $125 per milligram. This translates to about $100,000 (in U.S. currency), per *gram* of ZX, *in a racemic mixture.* Clearly, these preparations have no realistic potential for use as drugs or nutritional supplements, both because of their price, and because they contain large quantities of the undesired and possibly dangerous S-S and meso isomers. Prior to this invention, purified or semi-purified R-R zeaxanthin simply is not available to the public, in any form.

Prior art items which describe ZX production using microbial fermentation include:
(1) Courington and Goodwin 1955, the earliest known reference describing ZX production by bacteria from the genus *Flavobacter.*
(2) US patent 3,891,504 (Schocher and Wiss, 1975, assigned to Hoffman LaRoche), also describing ZX production by *Flavobacter* cells. These cells containing ZX were fed to chickens, and caused suitable coloration.
(3) US patent 3,841,967 (Dasek et al, 1974) and US patent 3,951,743 (Shepherd et al, 1976). Both are assigned to Nestle. These describe methods and nutrients that could be used to increase the quantity of ZX produced by the bacteria.
(4) Two more recent US patents (US 5,308,759 and 5,427,783, both invented by Gierhart). applicant herein. These patents describe a strain of bacteria (*Flavobacterium multivorum*) isolated from a Missouri waterway. These bacteria were discovered to create ZX without creating substantial quantities of other carotenoids. This was important in the context of poultry and fish feed to give meat and yolks a darker color, because carotenoids compete against each other for uptake into the bloodstream, after being eaten by an animal. Accordingly, the absence of other carotenoids from the *F. multivorum* strain identified by Gierhart could make more ZX available as a pigment for the animal tissue, and would therefore increase its potency and performance.

The '759 patent claims methods for producing ZX for poultry or fish feed, using the strain of *F*. *multivorum* bacteria. The '783 patent, a divisional, claims the feed mixtures. Both patents are limited to the use of ZX in poultry or fish feed, and neither suggests anything about using ZX for treating humans.

Neither of the Gierhart patents make any statement about specific stereoisomers of ZX, for two reasons: (1) the status of the ZX stereoisomers generated by the *F. multivorum* cells was not known in 1989, when the applications were filed, and (2) since the patents were solely concerned with creating ZX for use in poultry or fish feeds, there was no apparent reason for concern over different stereoisomers.

The wild-type strain of *F. multivorum* was deposited with the ATCC, and was given ATCC accession number 55238. Because these bacteria generate a certain type of lipid called sphingolipids, the ATCC reclassified these bacteria as *Sphingobacterium multivorum,* and listed these cells under that name in their catalog. The *Sphingobacterium* name that appears in the ATCC catalog has not yet appeared in either of the reference works that are regarded as the official guides to microbial taxonomy: *Bergy's Manual of Systematic Bacteriology,* which is appended and updated by the *International Journal of Systematic Bacteriology.*

Efforts to create ZX by standard chemical synthesis (without using microbes) have been reported over the past 20 years, including US patents 4,153,615 (Saucy 1979), 4,952,716 (Lukac et al 1990), and 5,227,507 (Lukac et al 1993). However, these processes suffer from serious disadvantages. They typically require numerous reaction steps, and each step has a less-than-100% yield, so that the final yield of ZX at the end of the multi-step processing is relatively poor. In addition, chemical synthesis usually yields undesired S-S and S-R stereoisomers of ZX, as well as various conversion or degradation products, such as oxidized zeaxanthin, and zeaxanthin molecules that have lost one or more of the double bonds in the straight portion and/or end rings.

In summary, prior to this invention, there was no known source of purified R-R zeaxanthin suitable for human consumption, either as a drug or nutritional supplement.

As described below, the strain of *F. multivorum* (ATCC accession number 55238) and its mutagenized descendants may be used to generate the R-R stereoisomer of ZX as a sole detectable isomer, with no detectable quantities of the undesired S-S or S-R stereoisomers, for use in some embodiments of the present invention.

### SUMMARY OF THE INVENTION

According to a first aspect of the invention there is provided: the use of the 3R-3'R stereoisomer of zeaxanthin in the manufacture of a medicament for the treatment of macular degeneration in humans, wherein at least 90% of all zeaxanthin molecules in said medicament are the 3R-3'R stereoisomer of zeaxanthin, and the medicament is arranged in dosage form and contains at least 3 mg of the 3R-3'R stereoisomer of zeaxanthin per dosage.

In a second aspect there is provided: the use of the 3R-3'R stereoisomer of zeaxanthin in the manufacture of a medicament or nutritional supplement for the prophylaxis of macular degeneration in humans, wherein at least 90% of all zeaxanthin molecules in said medicament or nutritional supplement are the 3R-3'R stereoisomer of zeaxanthin, and the medicament or nutritional supplement is arranged in dosage form and contains at least 0.5 mg of the 3R-3'R stereoisomer of zeaxanthin per dosage.

The patient may, though need not necessarily, already have been diagnosed as suffering from macular degeneration. The patient may have elevated genetic susceptibility to macular degeneration and/or may be suffering from any of Stargardt's disease, Best's disease, Batten's disease, Sjogren-Larsson syndrome, cone-rod dystrophy, ovine ceroid lipofuscinosis or a disease involving lysosomal storage problems. In particular, the medicament or nutritional supplement may be for the treatment of age related macular degeneration and may be prophylactic - ie for reducing future risk of age related, or other, macular degeneration. Nutritional supplements containing the 3R-3'R stereoisomer of zeaxanthin in a form suitable for oral ingestion by humans are especially appropriate for reducing risk of macular degeneration.

In general, the medicament or nutritional supplement may include a diluent or carrier substance which is suited for administration to humans. In the case of nutritional supplements the diluent or carrier may be one suitable for oral ingestion by humans.

The medicament is, generally, arranged so that a sufficient quantity of the 3R-3'R stereoisomer may be administered to the patient to provide a therapeutic benefit in a human suffering from macular degeneration.

In a preferred embodiment of the first aspect of the invention zeaxanthin is used in the manufacture of a medicament in a unit dosage form which contains at least 10mg of the 3R-3'R stereoisomer of zeaxanthin. Where zeaxanthin is employed for reducing future risk of age-related, or other, macular degeneration in humans, in accordance with the second aspect, it may, for example, be used in the manufacture of a nutritional supplement e.g. for occasional or periodic oral ingestion, which supplement contains at least 0.5mg of the 3R-3'R stereoisomer of zeaxanthin in each dosage.

Dosage forms manufactured in accordance with the first or second aspect of the invention preferably contain a physiologically acceptable excipient, diluent or carrier. By "physiologically acceptable", it is particularly intended that the excipient, diluent, or carrier be suited to administration to humans. In a preferred embodiment of composition the diluent or carrier is suitable or intended for oral ingestion by humans, and the composition contains the 3R-3'R stereoisomer of zeaxanthin in an amount which is therapeutically effective for use in the treatment or prevention of macular degeneration in humans.

The 3R-3'R stereoisomer of zeaxanthin constitutes at least 90 percent of total zeaxanthin, particularly preferably at least 95%, and the S-S and S-R stereoisomers constitute less than 10 percent, particularly less than 5 percent of total zeaxanthin in the dosage form. In particular, the dosage form may comprise substantially no S-S and R-S stereoisomers. For example, 3R-3'R zeaxanthin may be the sole detectable stereoisomer of zeaxanthin present.

It is also preferable that 3R-3'R zeaxanthin constitutes at least 90 percent of total carotenoids in the dosage form. This is preferable since other carotenoids may compete against zeaxanthin for alimentary uptake or tissue deposition after ingestion.

In some embodiments the 3R-3'R stereoisomer of zeaxanthin has a protective coating for reducing degradation of the zeaxanthin by stomach acid.

Preferred embodiments of the dosage form manufactured according to the first or second aspect comprise at least 1%, preferably at least 2%, and particularly preferably 5% by weight zeaxanthin (desirably 3R-3'R zeaxanthin).

In some embodiments, the dosage form produced may contain non-pathogenic microbial cells which contain the 3R-3'R stereoisomer of zeaxanthin at a concentration of at least 2% by weight as a fraction of microbial cell mass.

where the dosage form is intended for use as a nutritional supplement it may desirably incorporate a carrier selected from food substances suitable or intended for oral ingestion by humans.

The 3R-3'R stereoisomer of zeaxanthin employed in the first or second aspect of the invention may be produced by culturing, in a liquid nutrient medium, under conditions which promote zeaxanthin synthesis, cells which synthesize the 3R-3'R stereoisomer of zeaxanthin at a level of at least 90 percent of all carotenoid molecules synthesized by the cells. The cells are preferably ones which synthesize this stereoisomer as sole detectable isomer. They may be bacterial cells descended from Flavobacterium multivorum strain ATCC no 55238. Alternatively, the cells may have been genetically engineered to contain at least one zeaxanthin synthesis gene containing a DNA sequence obtained from cells descended from a strain of Flavobacterium multivorum strain ATCC no 55238.

Bacterial cells of the strain of *Flavobacterium multivorum* cells (ATCC accession number 55238) do not create any detectable quantities of undesired S-S or S-R stereoisomers, and they do not synthesize significant quantities of other carotenoids such as β-carotene or lutein, which might compete against ZX for alimentary uptake after oral ingestion. After synthesis using these bacteria, the ZX can be purified by methods such as solvent extraction, and can be taken orally, either as a therapeutic drug by patients suffering from macular degeneration, or as a nutritional supplement by anyone who wants to reduce his or her risk of suffering from age-related macular degeneration, which is widespread among people over the age of about 50 or 60. Convenient ingestible formulations are also disclosed, such as (1) watertight capsules containing R-R zeaxanthin mixed with a carrier such as vegetable oil; (2) various foods (such as margarine, dairy products, syrup, cookie dough, and meat preparations that are not subject to harsh cooking) that contain R-R zeaxanthin as an additive; and (3) granular formulations that can be added to soups, salads, drinks, or other foods.

In a further aspect the invention provides a tablet or capsule comprising the 3R-3'R stereoisomer of zeaxanthin, and a physiologically acceptable carrier, wherein the 3R-3'R stereoisomer of zeaxanthin constitutes at least 90 percent of all zeaxanthin in the tablet or capsule.

Preferably, the tablet or capsule of this aspect of the invention contains at least 3mg of the 3-3'R stereoisomer of zeaxanthin.

### BRIEF DESCRIPTION OF THE DRAWINGS

Preferred embodiments of the invention are described below by way of example only and with reference to the accompanying drawings of which:
FIGURE 1 depicts the molecular structures of beta-carotene, lutein, and zeaxanthin, showing the structure of all three carotenoids and the numbering system for the end rings. These structures are known in the prior art.

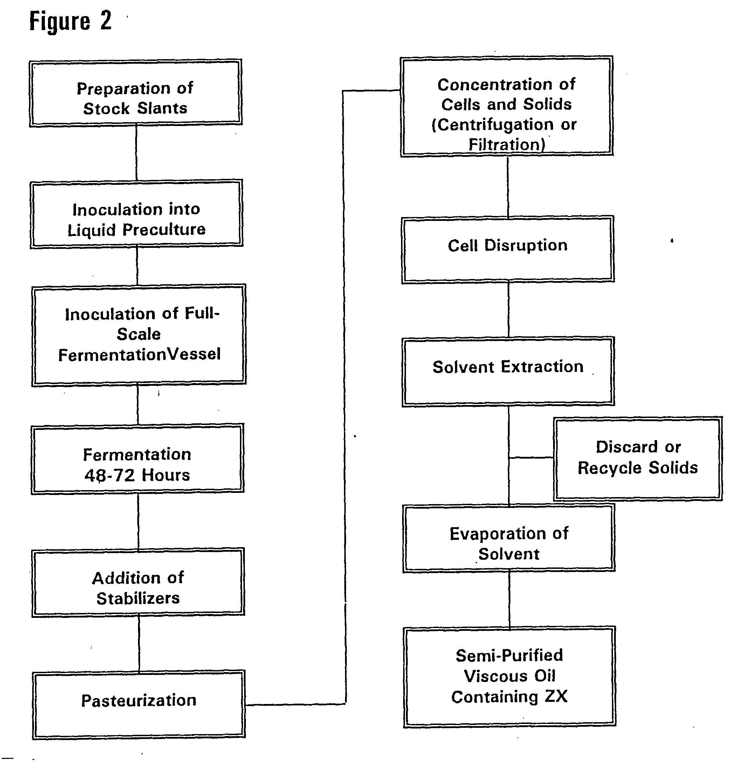

FIGURE 2 comprises a flow chart describing the steps in fermenting and purifying ZX generated by microbes that synthesize stereoisomerically pure 3R-3'R zeaxanthin.

### DESCRIPTION OF THE PREFERRED EMBODIMENTS

This invention discloses a method of manufacturing a medicament or nutritional formulation, for administration to humans, to prevent or reduce macular degeneration, a disease condition which damages the eyesight and can cause blindness. Zeaxanthin preparations intended for human ingestion should contain the 3R-3'R stereoisomer of ZX (also called the R-R isomer, or R-R zeaxanthin, for convenience) as a "heavily dominant" isomer. As defined herein, "heavily dominant isomer" refers to a preparation which contains at least 90% or more of the R-R isomer, with the undesired S-S or S-R isomers comprising less than 10% of all zeaxanthin in the preparation. Preferably, any preparations intended for human use should contain the R-R isomer of ZX as a sole detectable isomer, with no detectable quantities of the undesired S-S or S-R isomers. Such preparations are disclosed herein.

It has recently been discovered, using chiral column chromatography analysis (as described in Bone et al, 1993) that cells of the *F*. *multivorum* bacterial strain (ATCC accession number 55238) generate the R-R isomer as a sole detectable isomer of ZX, when fermented as described in the Examples. As described in Example 4, an analysis by Prof. Landrum indicated that there were no detectable quantities of either the S-S isomer or the R-S meso isomer, in ZX preparations created by fermenting cells from this *F. multivorum* strain.

Differences in the R-R, R-S, or S-S isomers of zeaxanthin will be very important if ZX preparations are administered to humans as a medicament or nutritional supplement, since the only ZX isomer that is naturally present in human retinas is the R-R isomer. It is believed that ingestion of significant quantities of the R-S and S-S isomers would be highly undesirable and dangerous, from a medical viewpoint, since (1) the R-S and S-S isomers do not naturally occur in human retinas, except possibly in extremely small trace quantities as byproducts that are formed when lutein is degraded inside retinal cells, and (2) the R-S and S-S isomers might competitively displace the desired R-R isomer in retinal tissue, possibly leading to serious long-term cellular damage and medical complications.

The stereoisomeric purity of ZX preparations made by fermenting the *F. multivorum* strain disclosed herein is extremely valuable, since it is extremely difficult and expensive to separate stereoisomers after ZX has been chemically synthesized. Although the separation of stereoisomers may be possible in small-scale laboratory settings, it is prohibitively expensive in commercial volumes.

### Use as a Prescription Drug for AMD Patients

In one aspect of this invention, the R-R zeaxanthin preparation disclosed herein can be formulated and administered as a drug, i.e., as a medicament which can be prescribed by physicians to treat patients who have been diagnosed as suffering from macular degeneration, or from a disease which can cause macular degeneration as a symptom or manifestation, such as Stargardt's disease, Best's disease, Batten's disease, Sjogren-Larsson syndrome, cone-rod dystrophy, ovine ceroid lipofuscinosis, or a lysosomal storage disease, such as Tay-Sach's disease.

When used for such treatment, a ZX preparation must have a sufficient amount of the R-R isomer of ZX to rise to the level of a therapeutic agent, in a carrier substance or form (such as a capsule) suited for administration to humans, as described below. In a preferred embodiment, ZX for medical treatment is packaged in unit dosage form, such as capsules or tablets. Each dose should contain at least 3 mg of R-R zeaxanthin, and may contain zeaxanthin in a range of 3 mg to 10 mg of zeaxanthin, if desired, for better therapeutic efficacy.

In another aspect of this invention, the R-R zeaxanthin preparation disclosed herein can be formulated and administered as a prophylactic medicament, for patients who have been diagnosed as having increased susceptibility to macular degeneration, such as due to a family history or genetic diagnosis of any of the diseases listed above. Unit dosages prepared for administration to such patients who are at elevated risk of AMD, but who do not yet suffer from actual AMD, may contain smaller quantities, such as 2 mg per dose.

Any of these dosages can be provided at commercially reasonable cost, using the disclosures herein. Capsules containing 25 milligrams (or any lesser quantity) in an oily carrier fluid can be created in an economical manner, using microbial fermentation coupled with a solvent extraction step. Powdered formulations containing even higher quantities (such as 100 milligrams or more per dosage) can also be created, if more extensive purification is used, such as the methods described in Example 4.

Examples of suitable ZX formulations include:
(a) a digestible watertight capsule and a fluid contained therein, wherein the capsule and fluid are sized and designed for oral ingestion by a human and are pharmacologically acceptable and wherein the fluid contains the 3R-3'R stereoisomer of zeaxanthin; preferably the zeaxanthin is enclosed within micelles created by a process using a bile salt; and
(b) a tablet designed for oral ingestion by a human, wherein the tablet contains the 3R-3'R stereoisomer of zeaxanthin and a compressible binder material which is compatible with zeaxanthin, and which causes a mixture of zeaxanthin and the binder material to retain its shape after compression under suitable pressure, and wherein the tablet is pharmacologically acceptable and properly sized for oral ingestion by a human; the tablet may have a digestible coating layer that helps protect the zeaxanthin against oxidation.

### Use as a Vitamin or Nutritional Supplement

In a still further aspect of this invention, ZX can be manufactured and packaged in the form of a vitamin or nutritional supplement or food additive, for consumption by people who do not currently suffer from macular degeneration, but who want to reduce their risk of macular degeneration later in life. When ingested for such purposes, appropriate dosages must be substantially higher than the trace quantities found in powders that are sold in health food stores today, but they nay be lower than when ZX is used as a therapeutic drug for people who have been diagnosed as suffering from AMD. Such dosages are likely to be in the range of 0.05 milligrams up to 5 mg, for a dosage to be ingested on a daily basis. For example, a dosage of 0.05 to 1.0 mg would be suitable when R-R zeaxanthin is one of a dozen or more agents in a multi-vitamin capsule or tablet, while a dosage of 1 to 5 mg can be made available for over-the-counter purchase by people who want higher dosages.

Examples of compositions containing ZX as a nutritional supplement include:
(c) a composition intended for oral ingestion by humans containing a food substance for human consumption (- generally one which is nutritionally acceptable and flavoursome-) which is suitable as a carrier for zeaxanthin, and the 3R-3'R stereoisomer of zeaxanthin added to the food substance as a nutritional additive; the food substance may be selected from margarine, dairy products, syrup, baked foodstuffs, cookie dough, brownie batter, meat preparations, and soup ingredients; preferably the zeaxanthin is microencapsulated and has a protective coating for reducing degradation of zeaxanthin by stomach acid; the food substance may comprise a granular formulation eg it may be a salt-containing flavouring mixture, spice-containing flavouring mixture, soup additive, baking mix or flavoured additive for milk; in such granular formulations the zeaxanthin may have a protective coating for reducing degradation of zeaxanthin by stomach acid;
(d) a composition comprising a foodstuff for oral consumption by humans, the foodstuff containing microbial cells, which may be intact, which are non-pathogenic (eg to humans), and which contain the 3R-3'R stereoisomer of zeaxanthin at a concentration of at least 2% zeaxanthin by weight as a fraction of microbial cell mass; the foodstuff may be selected from, cheese, yogurt, milk and beer; the microbial cells may have been killed by pasteurization.

Regardless of whether it is used as a therapeutic drug or nutritional supplement, a ZX preparation intended for human use should contain the R-R isomer as a sole or "heavily dominant stereoisomer" of ZX. The term "heavily dominant stereoisomer" is used herein to describe a ZX preparation in which the desired R-R isomer of ZX constitutes at least 90 percent of all ZX in the mixture, and the undesired S-S or S-R isomers constitute less than 10 percent.

Preferably, the R-R isomer should be the only detectable isomer of ZX in any preparation intended for human ingestion. This has been rendered feasible, in commercial volumes and at reasonable expense, by the because the *F. multivorum* bacterial line described herein generates the R-R isomer as a sole detectable stereoisomer of ZX. If any S-S or S-R isomers are present in the fermented mixtures after purification, their quantities are too small to be detected by the methods described in Example 4.

In addition, unlike most bacterial strains, the *F. multivorum* cells described herein do not generate a mixture of carotenoids; these cells create R-R zeaxanthin as the only detectable carotenoid. Since ZX must compete against other carotenoids for alimentary uptake and tissue deposition, this may be useful for increasing ZX uptake and retinal deposition after oral ingestion, especially in cases where ZX is being used as a drug to treat diagnosed cases of macular degeneration.

### Commercial-Scale Manufacture by Bacterial Fermentation

As known to those skilled in the art, methods used to ferment bacteria in laboratory settings can be very expensive and difficult to control when converted into large manufacturing operations. Accordingly, the Applicants have developed improved nutrients and methods for commercial use with its *F. multivorum* cells. The improved nutrients and methods are much easier to work with, and much less expensive per gram of ZX produced, than the media and conditions previously disclosed in US patents 5,308,759 and 5,427,783. Preferred nutrients and conditions are described in Example 1.

After fermentation, one or more stabilizing compounds such as t-butyl hydroquinone should can be added to the cells, to prevent degradation of the ZX during purification. Stabilizers can be added while the cells are still in a fermentation vessel, before pasteurizing or other processing begins. Various candidate stabilizers have been tested by the Applicants. The best results obtained to date use a combination of stabilizers listed in Example 2.

After stabilizers are added, the bacteria can be pasteurized by heating to 55°C for 25 minutes, to kill the bacteria without damaging the ZX. The culture is then cooled to room temperature, and liquid is removed from the cells by mechanical means, such as cross-flow microfiltration. This can increase the concentration of cells and solids from an initial value of about 10% to a filtered concentration of about 60 to 80%, by volume. This creates a cell paste.

It is possible that the *F. multivorum* cells, intact and possibly in a viable state, may be suitable for direct ingestion by humans, comparable to other foods (cheese, yogurt, beer, etc.) which contain viable or killed-but-intact microbial cells. There is no known pathogenicity associated with the *F. multivorum* cells. They were isolated from a cold waterway, and since they are adapted to living in cold water, they cannot survive or reproduce well at temperatures in the human body. In addition, these cells do not have any known toxic constituents; they are gram-negative, and do not have the cell wall structures that characterize gram-positive bacteria. When fed directly to birds or fish, in the form of a cell paste, the bacteria cells appeared to be well-suited as delivery vehicles. The ZX was released when the cells were digested by the animals, and the ZX was absorbed into the bloodstream and deposited into various tissues (including retinas) at appropriate locations.

Accordingly, intact *F. multivorum* cells containing R-R zeaxanthin may be suitable for direct human consumption, if desired, in any of three forms: (1) intact viable form; (2) intact killed form, after pasteurization; or (3) a formulation in which the bacterial cells have been killed and their membranes disrupted, to break open the cells and render the ZX more accessible. This can be done by means such as sonication (using high-frequency sound waves), high pressure, or grinding. Alternately, this step can be skipped if a solvent extraction procedure is used that disrupts the cell membranes.

If desired, ZX preparation can include a cell rinsing step, in which leftover nutrients and waste metabolites are removed after fermentation, by flushing the cells with a solution containing any desired ingredients, such as stabilizers, preservatives, or flavoring agents.

### Purification

If desired, a cell paste (with either intact or disrupted cells) can be dried to further concentrate the cells and increase the ZX concentration in the dried mass. This can be done by mechanical means such as spray drying (using heat) or lyophilization (freeze-drying under a vacuum). If drying is used, the resulting solid residue is usually referred to as a dried biomass; it usually contains about 1 to 10% ZX by weight, along with other cell solids, residual solids from the fermentation medium, and the stabilizers described above.

Before or after (or instead of) disruption or drying, an extraction step can be carried out to concentrate the ZX, which accumulates mainly in cell membranes. Suitable solvents for extraction generally include polar organic solvents. The best solvent identified to date is tetrahydrofuran (THF), which aggressively attacks the cells and renders a separate membrane disruption step unnecessary. Although stirring was not necessary when THF was used in laboratory operations, stirring during the solvent mixing step would probably be needed in commercial manufacturing.

Other solvents have also been tested and will continue to be tested and evaluated, but none tested to date worked as well as THF. Non-ringed organic solvents tested to date (such as acetone and diethyl ether) have lower ZX solubility levels, while other solvents such as methanol, ethanol, and hexane have even lower ZX solubility levels.

The solvent is mixed with a cell paste or dried biomass, under conditions that cause the solvent to dissolve as much of the ZX as possible. The dissolved liquid fraction is then separated from the solids, using means such as centrifugation or filtering. The solids can be discarded, or used as feedstock for other processing steps (including repeated solvent extraction cycles, if desired). The liquid fraction is treated to remove the solvent, usually by evaporation. This leaves behind a viscous oil containing R-R zeaxanthin, along with other soluble components that were drawn out of the cell paste by the solvent. When THF was used in a single-pass extraction on cells containing 1 to 3% ZX by weight, and when the THF was subsequently removed by evaporation, the resulting fluid contained about 5% to about 20% ZX by weight.

Another type of solvent extraction that has shown good preliminary results involves the use of a supercritical liquid (i.e, a compound which is normally a gas at atmospheric pressures, but which becomes a liquid which acts as a solvent at elevated pressure). Carbon dioxide is the most widely used solvent in supercritical extraction, and commercial-scale CO₂ extraction systems are available. In such systems, liquified carbon dioxide is mixed with a cell paste or dried biomass inside a high-pressure reaction vessel. The liquid is then passed through a series of chambers that reduce the pressure in a step-wise manner. ZX precipitates out of solution at a fairly high pressure, so it can be collected during an early de-pressurizing step, while the large majority of impurities remain soluble in the carbon dioxide and will be carried away to other reaction chambers where the pressure is reduced further. The efficiency of supercritical solvent extraction can be further increased by using entraining agents (such as ethanol, propylene glycol, or ethyl acetate). Several of these entraining agents have been preliminarily tested, and have been shown to substantially increase the solubility of ZX in supercritical carbon dioxide.

Although carbon dioxide is used widely in supercritical extraction, other compounds (including various nitrogen or chlorofluorocarbon compounds) are also used. Any such solvent which goes through gaseous and liquid phases as a function of pressure can be tested, to determine whether it is suitable for purifying ZX from bacteria as described herein.

If desired, an oily fluid containing ZX, generated by solvent or supercritical extraction, can be mixed with a carrier substance such as vegetable oil, and then enclosed within a capsule designed for human ingestion, without requiring any further purification of the ZX. This will provide an economical method of making a semi-pure digestible form of R-R zeaxanthin available for human use, either as a drug for people suffering from macular degeneration, or as a nutritional supplement for people who want to reduce their risk of suffering macular degeneration later in life.

Alternately, R-R zeaxanthin in a semi-pure oily liquid can be purified further, to increase ZX concentration and remove any impurities. This can be done by methods such as (1) two-solvent systems which use a combination of two selected solvents; (2) adsorption on a substrate (such as a woven filter bed) that encourages crystallization of ZX; or (3) counter-current chromatography. A chromatography method used to purify ZX to about 98% purity is described in Example 4.

Purification techniques for other carotenoids are disclosed in US patents 5,382,714 (Khachik 1995) and 4,851,339 (Hills 1989). Because of their chemical similarities, any technique which can purify β-carotene or lutein is likely to give good results in purifying ZX.

### Modes of Administration

Oral ingestion is the preferred mode of administering ZX to humans for retinal protection, using ingestion modes such as daily or weekly capsules, or use of ZX-supplemented foods or food additives, as described below. Treatment does not require regular ingestion at fixed intervals (such as daily or weekly pills), but instead refers to occasional, intermittent ingestion which allows a reasonable period of time (such as one or more days, preferably less than a week) to elapse between dosages, to allow gradual deposition of small quantities of ZX in the macular tissue. As with any vitamin supplement, a single dosage may be beneficial, but a single dosage will not be as beneficial over a period of years as periodic small dosages. Studies on carotenoid uptake in mammals indicate that daily ingestion is preferable to weekly or other sporadic intake, due to "loading" factors that are manifested in blood concentrations.

Since uptake of carotenoids after oral ingestion tends to be relatively low, it may be desirable for patients with severe macular degeneration to use other forms of administration, such as intramuscular or intravenous injection, or implantation of a slow-release device. Injectable carrier formulations can include water, a buffering agent, and an organic compound having multiple hydroxyl groups, such as propylene glycol, dextran, or cyclodextrin compounds.

Various modes of packaging for oral ingestion can be used, so long it protects the ZX from oxidation and takes account of the oily nature of ZX. Examples of suitable compositions for oral ingestion include:
(a) a digestible watertight capsule and a fluid contained therein, wherein the capsule and fluid are sized to be swallowed intact, and are pharmacologically acceptable, and wherein the fluid contains R-R zeaxanthin mixed with a suitable carrier or diluent such as vegetable oil. If desired, the fluidized ZX can be micro-encapsulated or enclosed within micelles, as described in Examples 9 or 10, to help protect the ZX against degradation in the stomach. Such capsules can be made of relatively rigid, stiff material, or of pliable material as commonly used in capsules containing vitamin E. If the capsule is made of material which withstands the acidity of the stomach and is digested by enzymes in the intestines, the ZX can be protected against degradation in the stomach, and the bioavailability of the ZX can be increased. However, it is known that at least a portion of the ZX that enters the stomach as a constituent of chewed vegetable mass can pass through the stomach unharmed; therefore, it is not essential to protect ZX against stomach acidity, and the choice of capsule material will be an economic preference rather than a scientific imperative.
(b) a tablet for oral ingestion by a human, wherein the tablet contains R-R zeaxanthin and a compressible binder material which is compatible with zeaxanthin, and which causes the tablet to retain its shape after compression under suitable pressure, and wherein the tablet is pharmacologically acceptable and sized to be swallowed intact. If desired, the tablet may have a coating to help protect the ZX against stomach acidity.
(c) a composition comprising a food substance for human consumption, which is nutritionally acceptable and pleasant in taste, which is suitable as a carrier for zeaxanthin, and which contains R-R zeaxanthin as an additive. ZX is a yellow-orange pigment with the same generally hydrophobic characteristics as vegetable oil, shortening, and chicken fat; it is also similar to other carotenoid food colorings, such as β-carotene. Accordingly, it can be added as a nutritious coloring agent to various food substances, such as margarine, dairy products, syrup, baked foodstuffs, cookie dough, brownie batter, meat preparations that will not be subjected to harsh cooking conditions, and soup ingredients. Other suitable food substances may comprise granular formulations, such as salted or spiced flavoring mixtures used as additives for soups, salads, baking, etc. Granular formulations may have a protective coating if desired, to reduce degradation of ZX by stomach acid. Numerous items describe the use of β-carotene and other carotenoids as food colorings and nutritional additives; examples include Klaui et al 1970; Klaui and Bauernfeind 1981; Colombo and Gerber 1991, and U.S. patents 4,522,743 (Horn et al 1985), 5,180,747 (Matsuda et al 1993), 5,350,773 (Schweikert et al 1994), and 5,356,636 (Schneider et al 1994). Due to their chemical similarities, any method for adding β-carotene or lutein to food intended for humans is also likely to be directly applicable to R-R zeaxanthin.
(d) a composition comprising a foodstuff for oral consumption by humans, wherein the foodstuff contains microbial cells which are harmless to humans, and which contain the R-R isomer of zeaxanthin. Such foodstuffs may be selected from cheese, yogurt, milk and beer. The microbial cells may be viable if desired, or they may have been killed by methods such as pasteurization or fragmentation.

Other forms of packaging are also feasible and may be preferred for various uses.

### Testing of R-R Zeaxanthin in Animals

ZX that was synthesized using *F. multivorum* cells descended from the ATCC 55238 line was tested for retinal protection in a bird species, *Coturnix coturnix japonica,* commonly called the Japanese quail. This species provides a useful animal model of macular degeneration in humans, due to a number of factors, including:
(1) The entire retina of Japanese quails resembles the human macula in a number of important aspects. For example, the quail retina contains both ZX and lutein, and like a human macula, it is rich in photoreceptor cones rather than rods.
(2) The Japanese quail retina displays some of the same indicators of pathology as human retinas. For example, Japanese quail retinas accumulate soft drusen and lipofuscin, which are strongly correlated with onset of AMD in humans.
(3) Although quail retinas are much smaller than human retinas, the entire quail retina is colored yellow, due to the presence of ZX and lutein. This effectively allows the entire retina of a quail to serve as a model of the small macular region at the center of a human retina, and it renders analysis and observation much easier.
(4) The retina of the Japanese quail is avascular, and has a structure similar to the foveal region of the human retina.
(5) Japanese quail have a life-span of roughly 1 to 1.5 years for females, and 3 to 4 years for males. This allows studies of aging processes which would be very difficult in other species that live longer.

These factors are discussed in more detail in Fite et al 1991 and Fite et al 1993.

The tests are described in Examples 5 through 8. The results are excellent, and clearly indicate that R-R zeaxanthin produced by *F. multivorum* cells is (1) properly deposited in the macula after oral ingestion, and (2) highly effective in protecting retinal cells against phototoxic damage.

In addition, as discussed in Example 8, preliminary results indicate that R-R zeaxanthin is far more potent and effective than β-carotene in protecting retinas against phototoxic damage. When β-carotene was fed to test animals at a high dosage, the minor protective benefits provided by the β-carotene did not even reach a level of statistical significance. By contrast, when R-R zeaxanthin was fed to animals at the same dosage, it completely blocked and prevented the indicator of retinal damage that was being measured.

### Microbial Sources of R-R Zeaxanthin

*Flavobacterium multivorum* cells as disclosed herein were deposited with the ATCC (ATCC accession number 55238; as noted above, these are referred to as *Sphingobacterium multivorum* in the ATCC catalog, but their name has not been changed in the Bergy's Manual). This cell line provides those skilled in the art with several options for microbially synthesizing isomerically pure R-R zeaxanthin.

First, direct and unmodified descendants of these cells can be used to synthesize R-R zeaxanthin with no detectable quantities of other undesired stereoisomers. The total carotenoids generated by these cells comprise more than 90% ZX, the desired carotenoid.

Second, descendants of the ATCC 55238 strain can be used after they have been modified in ways which increase production of the R-R isomer of ZX. Mutant or other varied cell lines can be created by any of several methods, such as (1) treating descendants of the wild-type ATCC 55238 strain with mutagenic agents such as ultraviolet or X-ray radiation, or with known chemical mutagens such as N-methyl-N'-nitro-N-nitrosoguanidine; (2) generating sexual combinations, by mixing the *F. multivorum* cells with other types of bacteria that actively promote conjugation and exchange of DNA between bacterial cells; or (3) treating *F. multivorum* cells with bacterial transposons or viruses that can cause the rearrangement of relatively large chunks of DNA. These techniques introduce random alterations in descendant cells, and the descendants are analyzed by screening tests to identify and isolate offspring cells which produce higher levels of ZX.

The screening tests can be facilitated by using chemicals (such as diphenylamine, nicotine, or lovastatin) which suppress one or more enzymes involved in the biosynthetic pathway that generates ZX. In layman's terms, these suppressor drugs create hurdles or obstacles, which can be overcome only by mutant cells that produce abnormally high quantities of ZX. Fortunately, the tests which can be used to identify high-producing mutants or variants are simple, fast, and easy. Since ZX is a yellow pigment, simple visual observation of a culture plate can be used to identify mutant colonies which have the desired traits of (1) good cell growth rates, and (2) the ability to generate abnormally high quantities of the yellow pigment. If desired, automated equipment (such as automated plate readers, or cell sorting devices coupled to flow cytometers) can also be used in screening tests after treatment with a mutagen.

These mutagenic and screening techniques are conventional and well-known in this field of art. Any cells which are directly descended from the ATCC 55238 wild-type strain are regarded as descendants of these cells, even if they have been modified, mutagenized, or sexually combined with other cell lines in any of the ways listed above.

In a third alternate approach, non-descendant microbial cells can be created which contain genes that were isolated or derived from the ATCC 55238 cell line, which express enzymes that help synthesize R-R zeaxanthin. Such genes can be isolated and identified using known techniques. For example, DNA sequences from the carotenoid-producing "crt" genes listed in US patent 5,429,939 (Misawa et al 1995, discussed above) can be used as hybridization probes to search for carotenoid-producing genes having homologous DNA sequences, in the genome of the ATCC 55238 cell line. Carotenoid-producing genes isolated from these cells can then be inserted into plasmids, cosmids, phages, or other suitable vectors that can be used to genetically transform any desired type of host cell, such as *E. coli* cells, yeast cells, insect cells, or mammalian cells. This type of controllable genetic engineering will allow transformed cells to create R-R zeaxanthin, using genes obtained from the ATCC 55238 cells.

In addition, the protein-encoding portions of the ZX-producing genes from the ATCC 55238 cells (i.e., the portions of the genes that are transcribed into messenger RNA, which is subsequently translated into the enzymes that synthesize ZX) can be placed under the control of high-powered and/or inducible gene promoters. Such "chimeric" genes, containing gene promoters obtained from different genes, can be used for various purposes, such as (1) to suppress production of ZX while the cells are growing and reproducing, and then greatly increase production of ZX by the cells during fermentation; and (2) to insert the genes into new types of host cells that may be preferred for commercial use, such as *E. coli* cells or yeast cells, which can use well-known and highly optimized fermentation, handling, and purification techniques.

ZX-producing genes isolated from the ATCC 55238 cell line can also be enhanced by other well-known techniques. As one example, bacterial genes often use "non-preferred" codons, which reduce and control the quantity of a protein created by a gene. To relax this limiting mechanism, non-preferred codons in a ZX synthesizing gene from the ATCC 55238 cell line can be replaced by "preferred" codons which can increase expression of the ZX-producing enzyme in a selected host cell.

As another example, cysteine residues can hinder the activity or stability of an enzyme, by forming undesired disulfide bonds with other cysteine residues, either in the same or other protein molecules. Accordingly, the activity or stability of an enzyme can sometimes be increased by replacing one or more cysteine residues with other amino acid residues (e.g., US patent 4,737,462, by Mark). In addition, the expression of a protein can often be increased by inserting codons for common amino acids, such as glycine, in place of codons that code for methionine and tryptophan, which are less common and which tend to slow down and reduce the expression of a protein. After a synthetic gene is created which causes an amino acid substitution of this nature, the modified protein can be tested to determine whether it retains the desired enzymatic activity while being expressed in higher quantities or more stable form.

These are examples of known genetic engineering techniques that can be evaluated on ZX-producing genes isolated from the ATCC 55238 cell line, to determine whether any such modification will enhance the production of ZX by the *F. multivorum* cells, or by other types of host cells.

The phrase in the claims which refers to "cells which have been genetically engineered to contain at least one zeaxanthin-synthesis gene containing a DNA sequence obtained from a strain of *Flavobacterium multivorum* which has been given ATCC accession number 55238" includes cells containing genes having DNA sequences that were chemically synthesized, using a DNA or mRNA sequence determined by analyzing the ATCC 55238 cell line or descendants thereof. Automated DNA synthesis machines are well known and can be used to duplicate any known gene sequence, without requiring replication of the original host cell. A "zeaxanthin-synthesis gene" includes any gene which expresses an enzyme or other protein that is involved in the ZX biosynthesis pathway, and which can be used to increase ZX production if inserted into suitable host cells, regardless of which particular enzyme in the ZX biosynthetic pathway the gene encodes.

### EXAMPLES

### Example 1: Commercial-Scale Fermentation

The nutrient medium that was preferred by the Applicants for the initial small-scale laboratory testing of *Flavobacterium multivorum* was identified as nutrient medium E under Example 3 in US patents 5,308,759 (Gierhart 1994) and 5,427,783 (Gierhart 1995). This nutrient medium contained several ingredients that were expensive and difficult to work with. To reduce expense and inconvenience, substantial research to create a better commercial-scale nutrient medium was carried out after the initial filing date of those applications. The nutrient media that are currently preferred for commercial-scale fermentation have eliminated corn flour and several other ingredients. These preferred media contain either high maltose corn syrup or sugar beet molasses, at concentrations ranging from 1 to 10% w/v, along with corn steep liquor at 0.5 to 4% w/v; ammonium sulfate heptahydrate at 0.5% w/v; sodium chloride at 0.5% w/v; magnesium sulfate heptahydrate at 0.1% w/v; sodium acetate at 0.1% w/v; ferrous sulfate heptahydrate at 0.001% w/v; yeast extract at 0.2% w/v; thiamine-HCl at 0.01% w/v; between 1 and 6% w/v hydrolyzed casein (such as NZ Amine HD, sold by Sheffield Products, Division of Quest International, Norwich, NY); and vegetable oil at 1% v/v.

After these ingredients are mixed together, sufficient NaOH is added to raise the pH to 6.5; by contrast, when the nutrient medium was adjusted to pH 7.5, as described in the lab-scale experiments in US patents 5,308,759 and 5,427,783, too many solids precipitated out from the corn steep liquor.

The culture medium is sterilized by autoclaving at 121°C for 30 minutes, then it is cooled to 27°C and inoculated with 5 to 10% v/v of a "liquid preculture" containing a strain of *F. multivorum* which produces R-R zeaxanthin without producing the S-S or S-R stereoisomers.

The cells used to prepare a liquid preculture are maintained on a slant tube of plate count agar. These slant cultures are inoculated with clonal colonies of *F. multivorum* descended from the strain deposited by the Applicants with the ATCC (ATCC accession number 55238). After incubation for 48 hours at 28°C, stock slants are refrigerated at 4°C until use as inoculum for liquid media. Viable cells can also be frozen using conventional freezers, dry ice, or liquid nitrogen, for prolonged storage.

A liquid preculture is prepared using cells taken from an agar slant to inoculate 30 mL of liquid media prepared as described above, contained in a 300 mL baffled flask. The growth conditions are 28°C at pH 7.2 to 7.6, aerated by agitation at 250 RPM and cultivated for 24 hours. After an initial 24 hour incubation, the cells contained in one or more 30 mL preculture flasks are used to inoculate a ten-fold greater quantity of nutrient medium in a suitably sized fermentation vessel. The cells are then incubated for 48 to 72 hours at 28°C. The pH is maintained between 6.80 and 7.20 using NaOH and/or phosphoric acid. The dissolved oxygen level is kept at 30 to 40% of saturation by bubbling filtered air through the vessel at a rate of 1 volume of air per one volume of liquid per minute while agitating the vessel at 400 to 1000 RPM. Tests using periodic sampling and high performance liquid chromatography have indicated that maximum quantities of ZX will usually be produced within about 72 hours when the cells are fermented under these conditions.

### Example 2: Addition of Stabilizing Agents

ZX produced by the fermentation processes of Example 1 needs to be stabilized in order to facilitate subsequent purification and formulation, and to ensure purity. Stabilizing compounds can be added to the *F. multivorum* cells (or to a cellular extract containing ZX) at any time during a preparation or purification process; in general, one or more initial stabilizers should be added to the cells while they are still in the fermentation vessel.

Various candidate stabilizers have been tested by the Applicants. The best results obtained to date have used a combination of stabilizing agents, which are mixed together in a small quantity of a suitable solvent (such as about 2 milliliters of ethanol for a 20 liter fermentation vessel) before being added to the cells. The preferred stabilizer mixture contains tertiary butyl hydroquinone (abbreviated as TBHQ; also called 2-(1,1-dimethyl)-1,4-benzenediol) at a quantity which will generate a final concentration ranging from about 250 µ/L (micrograms per liter) up to about 50 mg/L after being mixed with the cells; ethoxyquin at a post-mixing concentration ranging from about 250 µg/L to about 250 mg/L; α-tocopherol at a concentration ranging from about 250 µg/L to about 250 mg/L; and EDTA (ethylene diamine tetra-acetic acid) at a concentration ranging from about 500 µg/L to about 500 mg/L. Suitable concentrations can vary widely, and will depend on various factors such as subsequent purification steps and the intended mode of packaging and ingestion. Preferred concentrations of these stabilizers, for use with single-pass THF extraction followed by mixing with vegetable oil and watertight encapsulation in a vitamin-type pill, are about 25 to 50 mg/L for TBHQ; 250 to 500 µg/L for ethoxyquin, 250 to 500 µg/L for α-tocopherol; and 500 to 1000 µg/L for EDTA.

After the stabilizers are added, the cell culture is pasteurized by heating to 55°C for 25 to 50 minutes. This kills the bacteria without damaging the ZX they have produced. The culture is then cooled to room temperature, and the ZX-containing cells and other solids present in the culture broth are separated from the liquid phase by means of a cross-flow microfiltration system which increases the cells/solids concentration from an initial value of about 10 to 15%, to a filtered concentration of about 60 to 80%, by volume. This procedure results in a cell paste, which also contains some residual solids from the nutrient medium.

For ZX preparations that are fed to Japanese quail for retinal testing, as described in Examples 5-7, the cell paste is frozen to -70°C, then dried by lyophilization at 25°C at full vacuum, to create a dried biomass containing about 1 to 10% ZX by weight. In past tests, the quantity of ZX in each batch was individually measured, and batches having different concentrations were combined and mixed together to ensure consistent concentrations for the Japanese quail tests.

To create ZX for human ingestion, solvent extraction is used to generate a viscous oily fluid, as described in Example 3.

### Example 3: Semi-Purification into an Oily Liquid

After a cell paste has been created as described in Example 2, it can be treated in any of a variety of ways. As mentioned above, the cell membranes can be disrupted, if desired, to break open the cells and render the ZX more accessible, by means such as sonication (high-frequency sound waves), high pressure, or grinding, keeping the temperature of the cells below about 30°C to prevent oxidation. However, this step has not been necessary when tetrahydrofuran (THF) is used in a solvent extraction step, since THF is very effective in disrupting the cell membranes without mechanical assistance. Stirring has not been necesary when THF is used in lab-scale operations; however it is likely that stirring during the solvent mixing step would probably be beneficial in commercial manufacturing operations.

In tests done to date, THF extraction involved mixing about 8 to 20 volumes of purified filtered THF with a volume of cell paste containing 60-80% solids, at a temperature below 25°C, for a period of 2 to 24 hours. The THF aggressively attacks the cells, creating a liquid with a suspension of flocculant solids. After centrifugation at up to 20,000 gravities for several minutes, most of the THF can be removed by decanting. The THF that remains can be evaporated under vacuum, to leave behind a viscous oil. When cell pastes containing 1 to 3% ZX were treated by THF extraction in a single-pass operation, the resulting oil usually contained about 5 to 20% ZX, by weight.

### Example 4: Preparation of Highly Purified Zeaxanthin in Dry Powdered Form, with 100% Pure R-R Isomer

A highly purified ZX preparation in dry powdered form was created by processing the THF-extracted oily fluid described in Example 3 by means of liquid chromatography, as follows. The oily ZX-containing liquid was dissolved in hexane, then passed through a chromatography column containing neutral alumina powder. Two column-volumes of hexane were used to wash the column, to remove carotenoid impurities such as ß-carotene and lycopene, as well as lipids and other contaminants. A mixture of hexane:acetone at 80:20 was then passed through the column, to release the ZX. The dissolved ZX which emerged was dried under vacuum. Chromatographic analysis indicated at least 98% pure ZX; only trace quantities of any impurities were detectable.

After roughly six months of storage in a relatively unprotected state (usually under normal refrigeration, with moderately frequent removal for sampling, and without containing any anti-oxidants and without taking any precautions to prevent contact by atmospheric oxygen), a sample of this ZX preparation was sent for stereoisomeric analysis to Prof. John Landrum (co-author of the Bone, Landrum, et al papers) at Florida International University in Miami, Florida. His analysis, using chiral column chromatography with dicarbamate derivatization, indicated that the six-month-old unprotected preparation contained 92% ZX. The impurities appeared to be mainly keto-carotenoids which pre-eluted before the ZX; keto-carotenoids have an extra oxygen atom attached somewhere to a carotenoid, and they are common by-products that arise when carotenoids are stored without being protected against oxidation. Prof. Landrum's chiral analysis indicated that 100% of the ZX in the preparation was the desired R-R isomer. There were no detectable quantities of the undesired S-S or S-R stereoisomers of ZX.

It is recognized that chromatography purification as described above, although entirely feasible and highly effective, is not ideally suited to preparing highly purified ZX in commercial quantities. An alternate method that was developed to purify lutein, described in US patent 5,382,714 (Khachik 1995; also see Khachik et al 1991), which uses a cold ethanol-water mixture in a two-solvent extraction system, followed by lyophilization, offers a good candidate method, for evaluation for commercial manufacturing use.

### Example 5: Tests of Zeaxanthin on Japanese Quail, Using Different Dietary Groups

All tests involving Japanese quail were carried out at the Schepens Eye Research Institute of Harvard Medical School (Boston, Massachusetts). All treatment or control groups contained statistically significant numbers of birds. In most cases, control groups were the same size as treatment groups.

Carotenoid-deficient bird feeds were obtained from Purina Mills (St. Louis, Missouri). These bird feeds are sold for experimental use only, and are obtained by using grain (such as milo seeds) that is naturally devoid of carotenoids.

All ZX preparations which were fed to the Japanese quail were in the form of dried biomass from *F. multivorum* cells that were fermented, stabilized with the agents described in Example 2, pasteurized to kill the cells, and dried using lyophilization.

All test animals were hatched from carotenoid-deficient eggs. These were created by feeding a parental generation (designated as P1 birds) with only carotenoid-deficient feed after the birds reached maturity. Their eggs were broken open and analyzed for carotenoids until the eggs became carotenoid-deficient. Eggs which were subsequently laid by carotenoid-deficient parental birds were used to hatch all test and control birds.

The test and control birds were divided into four major groups, which received different diets. These groups were designated as the C+ group, the C- group, the BC+ group, the ZX(+5) group, and the ZX(+50) group, depending on which carotenoids they received in their diets.

Birds in the C+ group were fed a standard commercial diet which contained several carotenoids; this diet also contains synthetic alpha-tocopherol (Vitamin E) as an additive.

Birds in the C- diet group were fed a diet which is essentially devoid of all carotenoids, as described above. However, this diet contained all other essential nutrients, and it contained synthetic vitamins A and E as additives.

Birds in the BC+ group received a diet devoid of all carotenoids except for β-carotene as an additive, at the same dosage that was used in the high-dosage ZX group (i.e., 50 mg of β-carotene was added, per kilogram of feed). Birds in the BC+ group were transferred to the diet containing β-carotene seven (7) days before the light damage procedure was initiated. This group allows a direct comparison of β-carotene and one sub-group of the ZX supplemented birds which were also transferred from a deficient diet to a ZX supplemented diet at seven days prior to light damage. As described in Example 8, this direct comparison showed that ZX is highly effective in preventing phototoxic damage, while β-carotene's protective effects were so weak that they failed to reach a level of statistical significance.

Birds in the ZX+ group received a diet devoid of all other carotenoids, but which contained dried biomass containing R-R zeaxanthin from *F. multivorum* cells. Two different dosages of ZX were fed to these birds in order to allow a dose-and-effect relationship to be evaluated and correlated with various indicators of retinal damage. Birds in the ZX(+5) group received a relatively small quantity of ZX, averaging 5 mg of added ZX per kilogram of feed. Since Japanese quail eat about 25 to 35 grams of food per day, about 0.125 to 0.175 milligrams of ZX were ingested per bird per day in the low dosage group. Birds in the ZX(+50) group received a ten-fold higher quantity (50 mg of ZX per kilogram of feed), causing these birds to consume an average of 1.25 to 1.75 milligrams of ZX per bird per day.

The carotenoid concentrations in the control, deficient, and ZX+ diets were analyzed by high performance liquid chromatography (HPLC), using the methods described in Stacewicz-Sapuntzakis et al 1993. The results are shown in Table 1.

**TABLE 1**

| Diet | Carotenoid Concentrations (µg/g) | | | | | |
|---|---|---|---|---|---|---|
| | ZX | Lutein | β-Cryp | β-Car | Canthax | Lycop |
| Control (C+) | 0.59 | 1.55* | 0.11 | 0.24 | 0.00 | 0.00 |
| Deficient (C-) | 0.26 | 0.59* | 0.00 | 0.00 | 0.00 | 0.00 |
| Zeaxanthin (ZX+50) | 67.60 | 0.59* | 1.90 | 3.00 | 0.00 | 2.90 |
| Zeaxanthin (ZX+5) | 6.74 | 0.06* | 0.20 | 0.28 | 0.00 | 0.27 |

| | | | | | | |
|---|---|---|---|---|---|---|
| KEY: ZX = 3R-3'R-Zeaxanthin β-Cryp = β-Cryptoxanthin β-Car = β-Carotene Canthax = Canthaxanthin Lycop = Lycopene * = Tentative Identification. This may also be a cis-isomer of zeaxanthin. | | | | | | |

All birds were raised and kept in normal brooding cages. Except as noted below, they were kept under normal broad-spectrum lighting, ranging from 10 to 14 hours per day.

### Example 6: Retinal Deposition of Orally Ingested Zeaxanthin

Chemical analyses were performed to determine the concentrations of ZX (and other carotenoids) that were deposited in the retinas of the birds in each of the various dietary groups described in Example 5.

To perform these analyses, birds that had been hatched from carotenoid-deficient eggs, and which had been raised on their respective diets for at least six months, were sacrificed by cervical dislocation. Retinal tissue was removed by dissection of the enucleated eye, and tissue from a single retina was ground to near-homogeneity in 250 *µ*L of distilled deionized water, using a glass or polytetrafluoroethylene (TEFLON™) pestle. 10 *µ*L of the homogenate was removed and used for protein analysis of the homogenate in order to normalize results from the various retinal samples. 250 *µ*L of methanol containing 2% w/v pyrogallol and 50 *µ*L of 60% w/v potassium hydroxide was added to the remaining 240 *µ*L of retinal tissue suspension. The mixture was heated in a water bath at 70°C for one hour, then 500 *µ*L of 50% v/v ethanol was added, followed by 2 mL hexane. The mixture was vortexed to mix it thoroughly, then allowed to stand at 5°C until separation occurred. The epiphase (i.e., the lighter phase which floated on top of the remaining liquid), containing hexane and extracted carotenoids, tocopherols, and retinols, was removed. 2 mL of additional hexane was added to the tissue homogenate, and the mixture was vortexed and allowed to separate again. This epiphase was removed and combined with the first. This procedure was repeated once more, in a third extraction cycle, to ensure complete extraction of carotenoids, tocopherols and retinoids.

The combined hexane extracts were then washed with 1 mL of water, to remove residual potassium hydroxide. 1 mL of additional hexane was added to the hexane-water mixture, then the hexane layer (epiphase) was carefully removed by pipetting. The hexane was then evaporated under a constant stream of nitrogen gas. The residue that remained contained carotenoids, tocopherols, retinols and other unidentified hexane-extractable compounds. This residue was then dissolved in solvent (methanol:chloroform:triethylamine) and analyzed by HPLC, as described above.

The results are listed in Table 2, below, which also shows damage levels after high-intensity light exposure.

**Table 2**

| Diet Type | Mean Values (n=6) | | | |
|---|---|---|---|---|
| | Retinal Carotenoid (ng/mg Protein) | | Number of Cone Apoptotic Nuclei in Central Retina (400X Magnification Field) | |
| | Zeaxanthin | Lutein | Non-Light Damaged | Light Damaged |
| Control (C+) | 30.18 | 10.41 | 0 | 60 |
| Deficient (C-) | 10.29 | 8.03 | 0 | 120 |
| β-Car (+50) | 10.01 | 7.83 | 0 | 109 |
| Zeaxanthin (ZX+5) | 31.00 | 3.64 | 0 | 15 |
| Zeaxanthin (ZX+50) | 104.17 | ND | 0 | 0 |
| ND = None detected in these samples. A lutein-like compound was detected, but this apparently was not lutein, as determined by HPLC retention time and photodiode array scanning of the peak in question. | | | | |

It should be noted that no β-carotene was detected in any retina taken from birds on any of the described diets.

These retinal concentrations listed in Table 2 indicate that the orally ingested R-R zeaxanthin, created by fermenting cells descended from *Flavobacterium* *multivorum* (ATCC accession number 55238), was indeed deposited in the retinas of the test animals that received the R-R zeaxanthin as a dietary supplement, in the form of a feed additive. These are important findings, since the ZX must be digested in a normal manner, it must cross the intestinal barrier, it must enter the bloodstream, and it must be taken up by retinal cells in the birds' eyes in sufficient quantities to protect retinal tissue against phototoxic damage. All of these hurdles were overcome by the bacterially fermented R-R zeaxanthin preparations described herein.

### Example 7: Protection of Retinas by R-R Zeaxanthin

Some of the birds in each dietary group were subjected to high-intensity visible light, at 2,000 to 3,000 lux, for a 28 hour period, using cycles comprising 1 hour of light followed by two hours of near-total darkness. Following the light-cycling period, the birds were placed in near-total darkness for a period of 14 hours before being sacrificed. This amount of high-intensity light exposure had been determined, in preliminary tests, to cause consistently severe damage in birds in the carotenoid deficient group, and moderate damage in birds in the control (normal diet) group. It was also determined in preliminary tests that the period of 14 hours after light exposure was necessary in order to measure the maximum number of apoptotic cone nuclei in unprotected (carotenoid deficient) birds.

Birds fed a control diet demonstrated maximum apoptosis at approximately 24 hours post-exposure, while birds fed a diet supplemented with ZX demonstrated maximum apoptosis at a time much greater than 24 hours. The extension of time before damage can be measured is, in and of itself, a strong indicator of the protective effects of ZX.

The retinas from these birds were isolated by microdissection, fixed in xylene and desiccated in ethanol before embedding in paraplast (Oxford, 56°C). The retinal tissues in paraplast were then sectioned and stained by the method of Gallyas 1990, or by propidium iodide to visualize the pyknotic nuclei, which characterize apoptosis. The number of pyknotic nuclei which could be seen in a single microscopic field at 400 X (linear) magnification were counted. The nuclei in at least 6 to 8 separate fields were counted for each treatment group, and the values were averaged.

The results, in Table 2, clearly demonstrate that retinal cell death and damage were: (1) greatly reduced and/or delayed by the bacterially-synthesized R-R zeaxanthin, even at the low ZX(+5) dosage level, compared to birds that received the normal control diet; (2) reduced even more by the higher ZX(+50) dosage. The complete absence of any pyknotic nuclei in retinas from the ZX(+50) treatment group offers compelling evidence that R-R zeaxanthin from the *F. multivorum* cell line (ATCC accession number 55238) offers a dramatic and extraordinary breakthrough in protecting retinal cells against phototoxic damage. To the best of the Applicants' knowledge and belief, no other agent ever tested has been able to reach or even approach this level of protection.

### Example 8: Direct Comparison of R-R Zeaxanthin Against β-Carotene in Protecting Retinal Tissue

As indicated above, birds in the BC+ dietary group were fed a dosage of β-carotene (50 mg, per kilogram of feed) that equalled the dosage of zeaxanthin in the ZX(+50) group. This allowed a direct comparison of β-carotene against ZX, in protecting retinal cells against phototoxic damage.

The results showed that phototoxic damage in the BC+ group was reduced by only a very small margin (about 10% or less, on average). Compared to the standard deviations in the control group, this reduction was not statistically significant; the probability that the small reductions were due solely to random fluctuations was 0.12 to 0.14.

This failure of β-carotene to offer better protection against phototoxic damage in retinal tissue, while R-R zeaxanthin at the same dosage offered a total 100% reduction in the same indicator of cell damage and death, starkly demonstrates the importance of this discovery. R-R zeaxanthin, synthesized by microbial fermentation, offers a major breakthrough which far surpasses any previously known agent.

### Example 9: Formation of Absorption Enhancers

Zeaxanthin-containing "micelles" which are less than 1 micron in diameter, and which contain only the desired R-R isomer of zeaxanthin, can be obtained from either the solvent extract of biomass or the oily fluid described in Example 3, by using certain types of bile salts, as described in Olson 1994. An oily fluid which contains R-R zeaxanthin can be mixed with a suitable bile salt, such as the phosphate salts of glyco- or taurocholate sold by Marcor Development Company of Hackensack, New Jersey or by use of gall bladder extracts containing mixtures of bile salts such as that sold by Salzman Corporation of Davenport, Iowa. This bile material can be mixed with either the solvent extract or oily mass and with certain other salts including sodium chloride, calcium chloride or potassium chloride. This mixture is then processed in a mechanical homogenizer which contains mixing devices such as rotating blades, at a rotation speed and for a duration which can be optimized by routine experimentation, by analyzing the micelle size ranges created by various combinations of blade sizes and shapes, rotation speed, and duration. The resulting micelles are dried free of solvent, if necessary, then diluted to any desired concentration using a carrier or diluent fluid such as vegetable oil. This mixture can then be enclosed within a capsule or other device that will aid in swallowing and help protect the resultant micelles against degradation by stomach acid.

Other emulsifiers and lipids can also be utilized to form emulsions with small particle sizes. Nonionic detergents such as Tweens and Spans can be utilized, as described in Olson 1994, as well as lipid materials such as phospholipids and sphingolipids, which would form lipid vessicles of small (less than 1 micron) size.

### Example 10: Micro-encapsulated Zeaxanthin

This example describes the preparation of zeaxanthin in micro-encapsulated form. Microcapsules are solid particles in the range of 10 to 1000 µm consisting of a core material (such as R-R zeaxanthin) encapsulated by a coating material or shell, can be prepared from different compounds such as gelatin, arabic gum, starch or zein (a protein from corn). During the preparation of the shell material, other compounds can also be added, to help maintain the shape, texture, stability, or other desired traits of the resulting preparation. Such compounds can include emulsifiers, sorbitol, antioxidants such as TBHQ or 2-[1,1-dimethyl]-1,4-benzenediol, or gelling agents such as carrageenan.

Pure or partially purified zeaxanthin is dissolved in an appropriate solvent, such as ethanol, acetone or THF. Once in solution, microcrystals of less than 10 microns in diameter are formed by adding the dissolved zeaxanthin into water. This process is improved if, during the addition, the water the zeaxanthin crystals in solvent are sonicated at high frequencies in the presence of emulsifying agents such as Tween 80.

Once the microcrystals are formed, the shell material is added to the mixture of water, zeaxanthin and solvent. With some shell materials, such as gelatin, it is necessary to maintain the mixture at temperatures as high as 60°C for up to two hours. Once the shell material is completely dissolved, the whole mixture is placed in a sonicator for a period of 5 to 10 minutes, in order to re-emulsify the crystals.

The formation of the microcapsules is accomplished by drying the mixture of core and shell material using any suitable drying method, such as spray drying, or use of a rotating disk by the method of Sparks et al, as described in US Patent No. 4,675,140. Spray driers are widely used in the food and feed industry. The rotating disk is an instrument which consist of a disk (approximately 4" diameter) that can be maintained at a certain temperature under controlled conditions. It can be operated at various speeds in the range of 1,000 to 10,000 revolutions per minute (rpm). A mixture of core and shell materials are added to the center of the disk while it is rotating at a speed such as 4000 rpm. The microcapsules are formed when the liquid contacts the heated rotating disk. Microcapsules are ejected away from the center of the disk by centrifugal force, and collected on a flat surface which has been pre-coated with a "collecting" or "catching" agent such as hydrophobic starch or dextrin. The microcapsules are then separated from the catching agent by size sieving. The microcapsules are placed in a container to protect them from the light and air and are stored under refrigerated conditions until measured into capsules for oral ingestion.

### REFERENCES

Bauernfeind, J.C. (ed.), *Carotenoids as Colorants and Vitamin A Precursors: Technological and Nutritional Applications* (Academic Press, New York, 1981)
Bone, R.A., "The role of the macular pigment in the detection of polarized light," *Vision Research 20:* 213-220 (1980)
Bone, R.A., et al, "Preliminary identification of the human macular pigment," *Vision Res. 25:* 1531-1535 (1985)
Bone, R.A., et al, "Analysis of the macular pigment by HPLC: retinal distribution and age study," *Invest. Opthalmol. Vis. Sci. 29:* 843-849 (1988)
Bone R.A., et al, "Stereochemistry of the macular carotenoids," *Invest. Ophthalmol. Vis. Sci. 34*: 2033-2040 (1993)
Bone, R.A., et al, "Distribution of macular pigment stereomers in individual eyes, including those with age-related macular degeneration (AMD)," *ARVO Abstracts: Invest. Ophthalmol. Vis. Sci. 35:* 1502 (1994)
di Mascio, P., et al, "Lycopene as the most efficient biological carotenoid singlet oxygen quencher," *Archives of Biochemistry and Biophysics 274*: 532-538 (1989)
Dorey, C.K., et al, "Lipofuscin in aged and AMD eyes," in *Retinal Degeneration* (Hollyfield et al, editors, Plenum Press, New York, 1993)
Eye Disease Case Control Study Group, "Antioxidant status and neovascular age-related macular degeneration," *Arch. Ophthalmol. 11:* 104-109 (1993)
Eye Disease Case Control Study Group, "Risk factors for neovascular age-related macular degeneration," *Arch. Ophthalmol. 10:* 1701-1708 (1992)
Foote, C.S., et al, "Chemistry of singlet oxygen. XI. Cis-Trans isomerization of carotenoids by singlet oxygen and a probable quenching mechanism," *J. Amer. Chem. Soc. 92:* 5218-5219 (1970)
Foote, C.S., et al, "Chemistry of singlet oxygen. X. Carotenoid quenching parallels biological protection," *J. Amer. Chem. Soc. 92*: 5216-5218 (1970)
Gallyas, F., *Acta Neuropathologica 79:* 620 (1990)
Gerster, H., "Review: antioxidant protection of the ageing macula," *Age and Aging 20:* 60-69 (1991)
Gittinger, J.W., *Manual of Clinical and Problem Ophthalmology* (Little-Brown, Boston, 1988)
Haegerstrom-Portnoy, G., "Short-wavelength-sensitive-cone sensitivity loss with aging: a protective role for macular pigment?," *J. Opt. Soc. Am. A5:* 2140-2144 (1988)
Ham, W.T.,Jr., et al, "Basic mechanisms underlying the production of photochemical lesions in the mammalian retina," *Current Eye Research 3:* 165-174 (1984)
Handelman, G.J. and Dratz, E.A., "The role of antioxidants in the retina and retinal pigment epithelium and the nature of prooxidant-induced damage," *Adv. in Free Radical Biology & Medicine 2:* 1-89 (1986)
Handelman, G.J., et al, "Carotenoids in the human macula and whole retina," *Invest. Ophthalmol. Vis. Sci. 29:* 850-855 (1988)
Jialal, I., et al, "β-Carotene inhibits the oxidative modification of low-density lipoprotein," *Biochimica et Biophysica Acta 1086:* 134-138 (1991)
Khachik, F., et al, "Separation, identification and quantification of carotenoids in fruits, vegetables and human plasma by high performance liquid chromatography," *Pure and Applied Chemistry 63:* 71-80 (1991)
Kirschfeld, K., "Carotenoid pigments: their possible role in protecting against photooxidation in eyes and photoreceptor cells," *Proc. R. Soc. Lond. B 216:* 71-85 (1982)
Malinow, M.R., et al, "Diet-related macular anomalies in monkeys," *Invest. Ophthalmol. Vis. Sci. 19:* 857-863 (1980)
Pease, P.L., et al, "Optical density of human macular pigment," *Vision Res. 27*: 705-710 (1987)
Peto, R., et al, "Can dietary beta-carotene materially reduce human cancer rates?" *Nature 290:* 201-208 (1981)
Schalch, W., "Carotenoids in the retina--a review of their possible role in preventing or limiting damage caused by light and oxygen," *EXS 62*: 280-298 (1992)
Seddon, J.M., et al, "Dietary carotenoids, vitamins A, C, and E, and advanced age-related macular degeneration, " *JAMA 272:* 1413-1420 (1994)
Snodderly, D.M., et al, "The macular pigment. I. Absorbance spectra, localization, and discrimination from other yellow pigments in primate retinas," *Invest. Ophthalmol. Vis. Sci. 25:* 660-673 (1984)
Sperduto, R.D., et al, "Do we have a nutritional treatment for age-related cataract or macular degeneration?," *Arch. Ophthalmol. 108:* 1403-1405 (1990)
Taylor, A., et al, "Oxidation and aging: impact on vision," *Journal of Toxicology and Industrial Health 9:* 349-371 (1993)
Vaughn, D. and Asbury, T., *General Ophthalmology,* 13th ed. (Appleton and Lange, Norwalk, CT, 1992)
Wald, G., "The photochemistry of vision," *Doc. Ophthalmol. 3:* 94 (1949)
Weiter, J.J., et al, "Central sparing in annular macular degeneration," *Am. J. Ophthalmol. 106:* 286-292 (1988)
Werner, J.S., et al, "Aging and human macular pigment density," *Vision Res. 27:* 257-268 (1987)

## Claims

1. Use of the 3R-3'R stereoisomer of zeaxanthin in the manufacture of a medicament for the treatment of macular degeneration in humans, wherein at least 90% of all zeaxanthin molecules in said medicament are the 3R-3'R stereoisomer of zeaxanthin, and the medicament is arranged in dosage form and contains at least 3 mg of the 3R-3'R stereoisomer of zeaxanthin per dosage.

2. Use of the 3R-3'R stereoisomer of zeaxanthin in the manufacture of a medicament or nutritional supplement for the prophylaxis of macular degeneration in humans, wherein at least 90% of all zeaxanthin molecules in said medicament or nutritional supplement are the 3R-3'R stereoisomer of zeaxanthin, and the medicament or nutritional supplement is arranged in dosage form and contains at least 0.5 mg of the 3R-3'R stereoisomer of zeaxanthin per dosage.

3. A use according to claim 1 or claim 2 wherein the dosage form is a capsule or tablet.

4. A use according to claim 3 wherein the dosage form is a capsule containing zeaxanthin microcapsules, the microcapsules having a size of 10 to 1000 µm and containing a core of R-R zeaxanthin encapsulated by a coating material.

5. A use according to any one of the preceding claims wherein the zeaxanthin is produced by a method which includes culturing, in a liquid medium, under conditions which promote zeaxanthin synthesis, cells which synthesize the 3R-3'R stereoisomer of zeaxanthin at a level of at least 90 percent of all carotenoid molecules synthesized by the cells.

6. A use according to any one of the preceding claims wherein the medicament or nutritional supplement is for the treatment or prophylaxis of macular degeneration in a patient suffering from Stargardt's disease, Best's disease, Batten's disease, Sjogren-Larsson syndrome, cone-rod dystrophy, ovine ceroid lipofuscinosis, a disease involving lysosomal storage problems, or having elevated genetic susceptibility to macular degeneration.

7. A use according to any one of the preceding claims wherein the 3R-3'R stereoisomer of zeaxanthin constitutes at least 90 percent of total carotenoids in the medicament.

8. A tablet or capsule comprising the 3R-3'R stereoisomer of zeaxanthin and a physiologically acceptable carrier, wherein the 3R-3'R stereoisomer of zeaxanthin constitutes at least 90 percent of all zeaxanthin in the tablet or capsule.

9. The tablet or capsule according to claim 8 which contains at least 3 mg of the 3R-3'R stereoisomer of zeaxanthin.

10. The tablet or capsule according to claim 8 or claim 9 wherein the 3R-3'R stereoisomer of zeaxanthin constitutes at least 90 percent of total carotenoids in the tablet or capsule.

11. The tablet or capsule according to any one of claims 8 to 10 for the treatment of macular degeneration in humans.

## Patentansprüche

1. Verwendung des 3R-3'R-Stereoisomers von Zeaxanthin bei der Herstellung eines Medikaments zur Behandlung von Makuladegeneration bei Menschen, worin zumindest 90 % aller Zeaxanthinmoleküle in diesem Medikament das 3R-3'R-Stereoisomer von Zeaxanthin sind und das Medikament in Dosierungsform vorliegt und zumindest 3 mg des 3R-3'R-Stereoisomers von Zeaxanthin pro Dosis enthält.

2. Verwendung des 3R-3'R-Stereoisomers von Zeaxanthin bei der Herstellung eines Medikaments oder einer Nahrungsmittelergänzung zur Vorbeugung gegen Makuladegeneration bei Menschen, worin zumindest 90 % aller Zeaxanthinmoleküle in diesem Medikament oder in dieser Nahrungsmittelergänzung das 3R-3'R-Stereoisomer von Zeaxanthin sind und das Medikament oder die Nahrungsmittelergänzung in Dosierungsform vorliegt und zumindest 0,5 mg des 3R-3'R-Stereoisomers von Zeaxanthin pro Dosis enthält.

3. Verwendung nach Anspruch 1 oder Anspruch 2, worin die Dosierungsform eine Kapsel oder Tablette ist.

4. Verwendung nach Anspruch 3, worin die Dosierungsform eine Kapsel ist, die Zeaxanthin-Mikrokapseln enthält, wobei die Mikrokapseln eine Größe von 10 bis 1.000 µm aufweisen und einen Kern aus R-R-Zeaxanthin enthalten, der von einem Beschichtungsmaterial eingekapselt ist.

5. Verwendung nach einem der vorangegangenen Ansprüche, worin das Zeaxanthin durch ein Verfahren hergestellt wird, das die Züchtung von Zellen, die das 3R-3'R-Stereoisomer von Zeaxanthin in einem Ausmaß von zumindest 90 % aller von den Zellen synthetisierten Carotinoidmoleküle synthetisieren, in einem flüssigen Medium unter Bedingungen, die die Zeaxanthinsynthese fördern, umfasst.

6. Verwendung nach einem der vorangegangenen Ansprüche, worin das Medikament oder die Nahrungsmittelergänzung zur Behandlung von oder Vorbeugung gegen Makuladegeneration bei einem Patienten dient, der am Stargardt-Syndrom, an der Best-Krankheit, am Batten-Syndrom, am Sjogren-Larsson-Syndrom, an Zapfen-Stäbchen-Dystrophie oder an oviner Ceroidlipofuscinose, einer Krankheit, die Speicherprobleme in Lysosomen mit sich bringt, leidet oder erhöhte genetische Anfälligkeit für Makuladegeneration aufweist.

7. Verwendung nach einem der vorangegangenen Ansprüche, worin das 3R-3'R-Stereoisomer von Zeaxanthin zumindest 90 % aller Carotinoide im Medikament ausmacht.

8. Tablette oder Kapsel, umfassend das 3R-3'R-Stereoisomer von Zeaxanthin und einen physiologisch annehmbaren Träger, worin das 3R-3'R-Stereoisomer von Zeaxanthin zumindest 90 % des gesamten Zeaxanthins in der Tablette oder Kapsel ausmacht.

9. Tablette oder Kapsel nach Anspruch 8, die zumindest 3 mg des 3R-3'R-Stereoisomers von Zeaxanthin enthält.

10. Tablette oder Kapsel nach Anspruch 8 oder Anspruch 9, worin das 3R-3'R-Stereoisomer von Zeaxanthin zumindest 90 % aller Carotinoide in der Tablette oder Kapsel ausmacht.

11. Tablette oder Kapsel nach einem der Ansprüche 8 bis 10 zur Behandlung von Makuladegeneration bei Menschen.

## Revendications

1. Utilisation du stéréoisomère 3R-3'R de la zéaxanthine dans la fabrication d'un médicament pour le traitement de la dégénérescence maculaire chez les humains, où au moins 90% de toutes les molécules de zéaxanthine dans ledit médicament sont le stéréoisomère 3R-3'R de zéaxanthine, et le médicament est arrangé sous forme de dosage et contient au moins 3 mg du stéréoisomère 3R-3R' de zéaxanthine par dosage.

2. Utilisation du stéréoisomère 3R-3'R de la zéaxanthine dans la fabrication d'un médicament ou complément nutritionnel pour la prophylaxie de la dégénérescence maculaire chez les humains, où au moins 90% de toutes les molécules de zéaxanthine dans ledit médicament ou complément nutritionnel sont le stéréoisomère 3R-3R' de zéaxanthine, et le médicament ou complément nutritionnel est arrangé en forme de dosage et contient au moins 0.5 mg du stéréoisomère 3R-3R' de zéaxanthine par dosage.

3. Utilisation selon la revendication 1 ou 2, où la forme de dosage est une capsule ou un comprimé.

4. Utilisation selon la revendication 3, où la forme de dosage est une capsule contenant des microcapsules de zéaxanthine, les microcapsules ayant une dimension de 10 à 1000 µm et contenant un coeur de R-R zéaxanthine encapsulé par une matière d'enrobage.

5. Utilisation selon l'une quelconque des revendications précédentes, où la zéaxanthine est produite par une méthode qui comprend la culture, dans un milieu liquide, dans des conditions qui favorisent la synthèse de la zéaxanthine, de cellules qui synthétisent le stéréoisomère 3R-3'R de la zéaxanthine à un niveau qui représente au moins 90% de toutes les molécules de caroténoïdes synthétisées par les cellules.

6. Utilisation selon l'une quelconque des revendications précédentes, où le médicament ou complément nutritionnel est pour le traitement ou la prophylaxie de la dégénérescence maculaire chez un patient souffrant de la maladie de Stargardt, la maladie de Best, la maladie de Batten, le syndrome de Sjogren-Larsson, la dystrophie cône-tige, la lipofuscinose céroïde ovine, une maladie impliquant des problèmes de stockage de lysosomes, ou ayant une sensibilité génétique élevée à la dégénérescence maculaire.

7. Utilisation selon l'une quelconque des revendications précédentes, où le stéréoisomère 3R-3'R de la zéaxanthine constitue au moins 90% des caroténoïdes totaux dans le médicament.

8. Comprimé ou capsule comprenant le stéréoisomère 3R-3'R de la zéaxanthine et un support physiologiquement acceptable, où le stéréoisomère 3R-3'R de la zéaxanthine constitue au moins 90% de toute la zéaxanthine dans le comprimé ou la capsule.

9. Comprimé ou capsule selon la revendication 8 qui contient au moins 3 mg du stéréoisomère 3R-3'R de la zéaxanthine.

10. Comprimé ou capsule selon la revendication 8 ou la revendication 9, où le stéréoisomère 3R-3'R de la zéaxanthine constitue au moins 90% des caroténoïdes totaux dans le comprimé ou la capsule.

11. Comprimé ou capsule selon l'une quelconque des revendications 8 à 10 pour le traitement de la dégénérescence maculaire chez les humains.
